# EUROPEAN PATENT SPECIFICATION

(11) **EP 1 309 617 B1**
(45) Date of publication and mention of the grant of the patent: **15.09.2010**
(21) Application number: 01956723.9
(22) Date of filing: 09.07.2001
(51) Int. Cl.: C07K 14/16, C12N 7/00, C12N 7/02

(54) **PROCESS FOR THE SELECTION OF HIV-1 SUBTYPE C ISOLATES, SELECTED HIV-1 SUBTYPE ISOLATES, THEIR GENES AND MODIFICATIONS AND DERIVATIVES THEREOF**
VERFAHREN ZUR SELEKTION VON HIV-1 SUBTYP C-ISOLATEN, SOLCHE ISOLATEN, SOWIE DEREN GENE, MODIFIKATIONEN UND ABLEITUNGEN
PROCEDE DE SELECTION D'ISOLATS DE SOUS-TYPE C DU VIH-1, ISOLATS DE SOUS-TYPE C DU VIH-1 SELECTIONNES, GENES, MODIFICATIONS ET DERIVES

(30) Priority: 07.07.2000 US 216995 P; 10.07.2000 ZA 200003437; 15.09.2000 ZA 200004924
(43) Date of publication of application: 14.05.2003
(73) Proprietor: MEDICAL RESEARCH COUNCIL, 7500 Cape Town (ZA); University of Cape Town, 7500 Cape Town (ZA); UNIVERSITY OF NORTH CAROLINA AT CHAPEL HILL, Chapel Hill, NC 27599-4100 (US); Alphavax, Inc., Research Triangle Park, NC 27709-0307 (US)
(72) Inventor: WILLIAMSON, Carolyn, 7500 Cape Town (ZA); SWANSTROM, Ronald, Ivar, Chapel Hill, NC 27599-4100 (US); MORRIS, Lynn Nat. Inst. for Communicable Diseases, 2131 Sandringham (ZA); KARIM, Salim, Abdool, University of Natal, Durban 4001 (ZA); JOHNSTON, Robert, Edward, Chapel Hill,NC 27599-410 0 (US)
(74) Representative: Albutt, Jodie
(86) International application number: PCT/IB2001/001208
(87) International publication number: WO 2002/004494

(56) References cited:
- DE BAAR M.P. ET AL.: "Subtype-specific sequence variation of the HIV type 1 long terminal repeat and primer-binding site" AIDS RES. AND HUMAN RETROVIR., vol. 16, no. 5, 20 March 2000 (2000-03-20), XP002221002
- TSCHERNING C. ET AL.: "Differences in chemokine coreceptor usage between genetyc subtypes of HIV-1" VIROLOGY, vol. 241, 1998, pages 181-188, XP002221003
- NOVITSKY VA ET AL: "Molecular cloning and phylogenetic analysis of human immunodeficiency virus type 1 subtype C: a set of 23 full-length clones from Botswana" JOURNAL OF VIROLOGY, THE AMERICAN SOCIETY FOR MICROBIOLOGY, US, vol. 73, no. 5, May 1999 (1999-05), pages 4427-4432, XP002144689 ISSN: 0022-538X -& DATABASE EMBL PROTEINS [Online] 1 November 1999 (1999-11-01) "Gag polyprotein" retrieved from EMBL Database accession no. Q9WF90 XP002221006 -& DATABASE EMBL PROTEINS [Online] 1 November 1999 (1999-11-01) "Gag-pol polyprotein" retrieved from EMBL Database accession no. Q9WF89 XP002221007 -& DATABASE EMBL DNA [Online] 11 March 1999 (1999-03-11) "HIV-1 isolate C-96BW04.02" retrieved from EMBL Database accession no. AF110962 XP002221008 -& DATABASE EMBL DNA [Online] 11 March 1999 (1999-03-11) "HIV-1 isolate C-96BW11.04 country Botswana" retrieved from EMBL Database accession no. AF110
- GAO F ET AL: "Molecular cloning and analysis of functional envelope genes from human immunodeficiency virus type 1 sequence subtypes A through G. The WHO and NIAID networks for HIV isolation and characterization" JOURNAL OF VIROLOGY, THE AMERICAN SOCIETY FOR MICROBIOLOGY, US, vol. 70, no. 3, March 1996 (1996-03), pages 1651-1667, XP002123321 ISSN: 0022-538X -& DATABASE EMBL PROTEINS [Online] 1 November 1996 (1996-11-01) "Envelope glycoprotein" retrieved from EMBL Database accession no. Q70014 XP002221011
- LOLE K S ET AL: "FULL-LENGTH HUMAN IMMUNODEFICIENCY VIRUS TYPE 1 GENOMES FROM SUBTYPE C-INFECTED SEROCONVERTERS IN INDIA, WITH EVIDENCE OF INTERSUBTYPE RECOMBINATION" JOURNAL OF VIROLOGY, THE AMERICAN SOCIETY FOR MICROBIOLOGY, US, vol. 73, no. 1, January 1999 (1999-01), pages 152-160, XP002929279 ISSN: 0022-538X -& DATABASE EMBL PROTEINS [Online] 1 November 1998 (1998-11-01) "envelope protein" retrieved from EMBL Database accession no. O90096 XP002221012
- LEIGH BROWN A.J. ET AL.: "Reduced susceptibility of human immunodeficiency virus type 1 (HIV-1) from patients with primary HIV infection to nonnucleoside reverse transcriptase inhibitors is associated with variation at novel amino acid sites." J. VIROL., vol. 74, no. 22, November 2000 (2000-11), pages 10269-10273, XP002221004 -& DATABASE EMBL PROTEINS [Online] 1 June 2001 (2001-06-01) "Reverse transcriptase" retrieved from EMBL Database accession no. Q99FC3 XP002221013
- DATABASE EMBL DNA [Online] 2 January 1996 (1996-01-02) "HIV-1 isolate BU/91/07, envelope" retrieved from EMBL Database accession no. HI1U39249 XP002221014
- VAN HARMELEN J.H. ET AL.: "A predominantly HIV Type 1 subtype C-restricted epidemic in South African urban populations" AIDS RES. AND HUMAN RETROVIR., vol. 15, no. 4, 1999, pages 395-398, XP002221005

## Description

### BACKGROUND TO THE INVENTION

THIS invention relates to a process for the selection of HIV-1 subtype (clade) C isolates, selected HIV-1 subtype C isolates, their genes and modifications and derivatives thereof for use in prophylactic and therapeutic vaccines to produce proteins and polypeptides for the purpose of eliciting protection against HIV infection or disease.

The disease acquired immunodeficiency syndrome (AIDS) is caused by human immunodeficiency virus (HIV). Over 34 million people worldwide are thought to be living with HIV/AIDS, with over 90% of infected people living in developing countries (UNAIDS, 1999). It is estimated that 24 million infected people reside in sub-Saharan Africa and that South Africa currently has one of the world's fastest growing HIV-1 epidemics. At the end of 1999, over 22 % of pregnant women attending government antenatal clinics in South Africa were HIV positive (Department of Health, 2000). A preventative vaccine is considered to be the only feasible way to control this epidemic in the long term.

HIV shows remarkable genetic diversity that has confounded the development of a vaccine. The molecular basis of variation resides in the viral enzyme reverse transcriptase which not only introduces an error every round of replication, but also promotes recombination between viral RNAs. Based on phylogenetic analysis of sequences, HIV has been classified into a number of groups: the M (major group) which comprises subtypes A to H and K, the O (outlier group) and the N (non-M, non-O group). Recently recombinant viruses have been more frequently identified and there are a number which have spread significantly and established epidemics (circulating recombinant forms or CRF) such as subtype A/G recombinant in West Africa, and CRF A/E recombinant in Thailand (Robertson *et al*., 2000).

Subtype C predominates in the Southern African region which includes Botswana, Zimbabwe, Zambia, Malawi, Mozambique and South Africa. In addition, increasing numbers of subtype C infections are being detected in the Southern region of Tanzania. This subtype also predominates in Ethiopia and India and is becoming more important in China.

A possible further obstacle to vaccine development is that the biological properties of HIV change as disease progresses. HIV requires two receptors to infect cells, the CD4 and co-receptors of which CCR5 and CXCR4 are the major co-receptors used by HIV-1 strains. The most commonly transmitted phenotype is non-syncytium inducing (NSI), macrophage-tropic viruses that utilise the CCR5 co-receptor for entry (R5 viruses). Langerhans cells in the mucosa are thought to selectively pick up R5 variants at the portal of entry and transport them to the lymph nodes where they undergo replication and expansion. As the infection progresses, viruses evolve that have increased replicative capacity and the ability to grow in T cell lines. These syncytium-inducing (SI) T-tropic viruses use CXCR4 in conjunction with or in preference to CCR5, and in some cases also use other minor co-receptors (Connor *et al*., 1997, Richman & Bozzette, 1994). However HIV-1 subtype C viruses appear to be unusual in that they do not readily undergo this phenotypic switch, as R5 viruses are also predominant in patients with advanced AIDS (Bjorndal *et al.,* 1999, Peeters *et al.,* 1999, Ping *et al*., 1999, Tscherning *et al.,* 1998, Scarlatti *et al*., 1997).

### SUMMARY OF THE INVENTION

The invention concerns a

A process for the selection of HIV subtype isolates for use in a vaccine comprising the following steps:
(a) isolating viruses from recently infected subjects;
(b) comparing the sequence, or part of the sequence, of at least one HIV gene of the isolated viruses, and generating a consensus amino acid sequence by identifying the most common codon or amino acid among the isolated viruses at each position along said sequence;
(c) selecting among all the isolated viruses the strains that fulfill the following criteria:
   (i) that the strain be genotypically representative of the circulating strains;
   (ii) that the strain not be an outlier strain;
   (iii) that the strain be as close as possible to the consensus amino acid sequence of (b);
   (iv) that the stain have an R5 phenotype, ie a phenotype associated with
      transmission for selection of the RNA or cDNA to be included for the *env* region; and
   (v) that the strain be able to be grown in tissue culture.

The HIV subtype is preferably a HIV-1 subtype C. which is non syncitium-inducing (NSI) and which typically utilises the CCR5 co-receptor for transmission. Examples of consensus sequences useful to carry out the invention are disclosed as chosen to carry out

By carrying out the process of the invention, three isolates were identified:
● HIV-1 subtype C isolate Du422, which was assigned accession number 01032114 by the European Collection of Cell Culture;
● HIV-1 subtype C isolate Du151, which was assigned accession number 00072724 by the European Collection of Cell Culture;
● HIV-1 subtype C isolate. Du179, which was assigned accession number 00072725 by the European Collection of Cell Culture.

Further isolates of interest will have the same DNA sequence (or a corresponding RNA sequence) of the *gag* gene of isolate Du422, of the pot gene of isolate Du151, or of the env gene of isolate Du151. Preferably, such isolates will have a sequence set out in any one of SEQ ID NOs 1 to 9.

These isolates, or their nucleic acids, can be used to prepare pharmaceutical compositions and vaccines.

Specific embodiments failing within the scope of the invention are defined in the appended claims.

### DESCRIPTION OF THE DRAWINGS

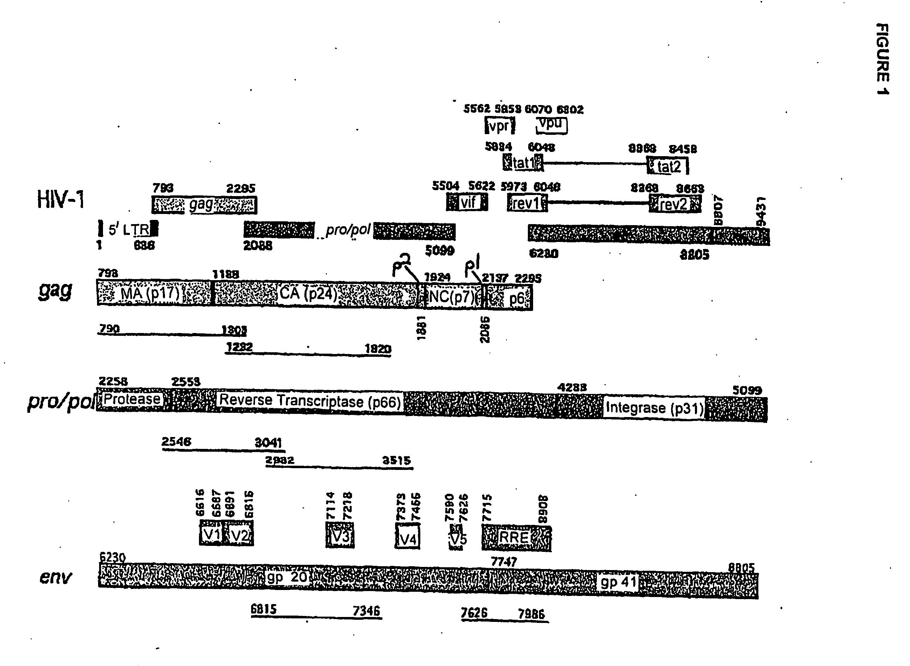

- **Figure 1**: shows a schematic representation of the HIV-1 genome and illustrates the location of overlapping fragments that were sequenced having been generated by reverse transcriptase followed by polymerase chain reaction, in order to generate the South African consensus sequence;

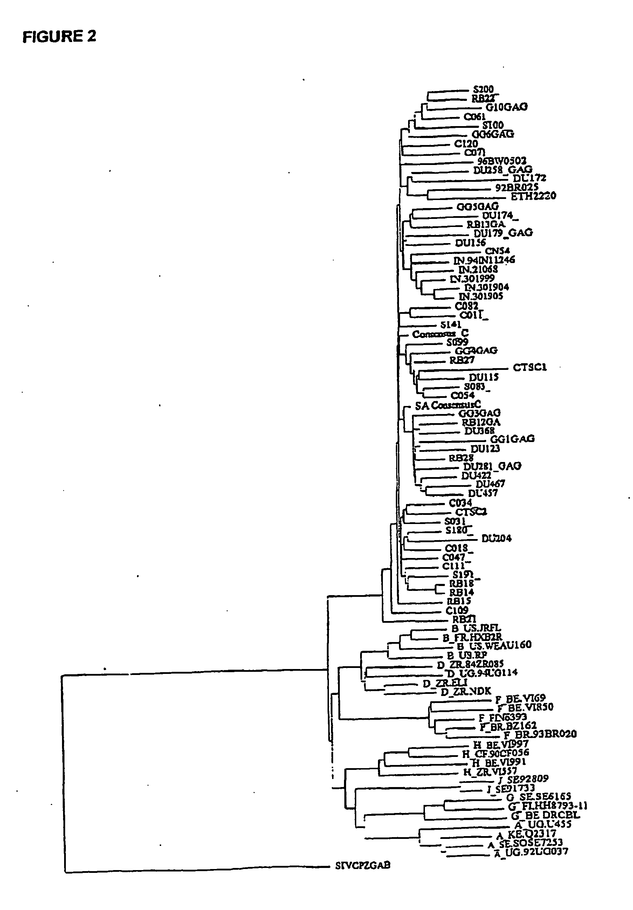

- **Figure 2**: shows a phylogenetic tree of nucleic acid sequences of various HIV-1 subtype C isolates based on the (partial) sequences of the *gag* gene of the various isolates and includes a number of consensus sequences as well as the South African consensus sequence disclosed herein and a selected isolate, Du422.

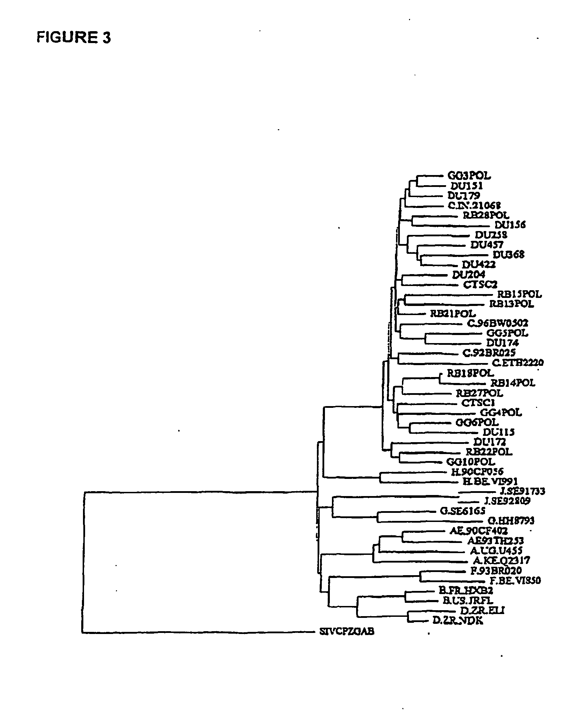

- **Figure 3**: shows a phylogenetic tree of nucleic acid sequences of various HIV-1 subtype C isolates based on the (partial) sequences of the *pol* gene of the various isolates and includes a number of consensus sequences as well as the South African consensus sequence **disclosed herein** and a selected isolate, Du151.

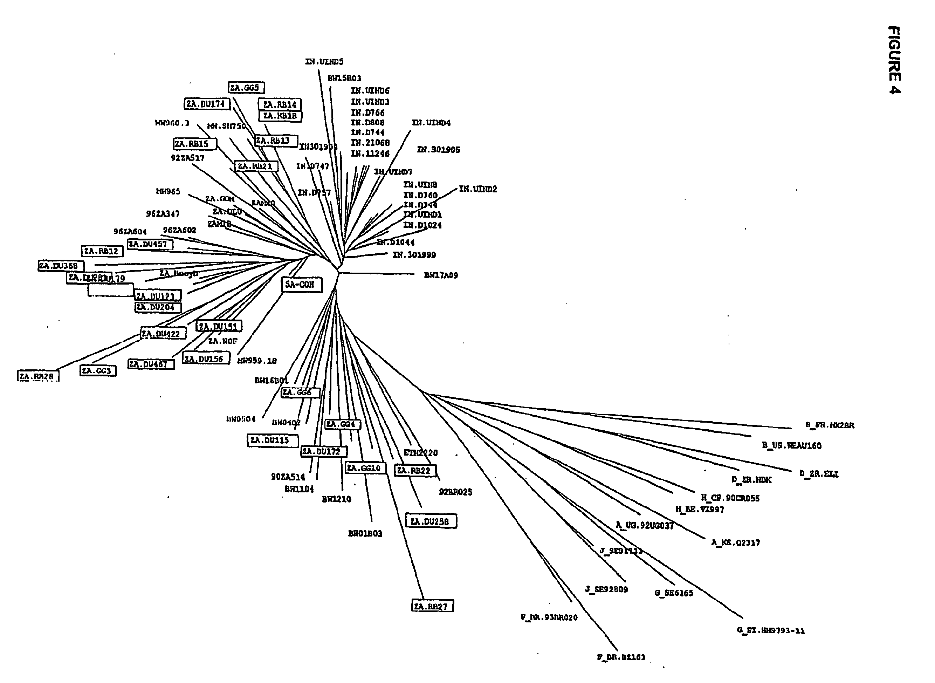

- **Figure 4**: shows a phylogenetic tree of nucleic acid sequences of various HIV-1 subtype C isolates based on the (partial) sequences of the *env* gene of the various isolates and includes a number of consensus sequences as well as the South African consensus sequence **disclosed herein** and a selected isolate, Du151.

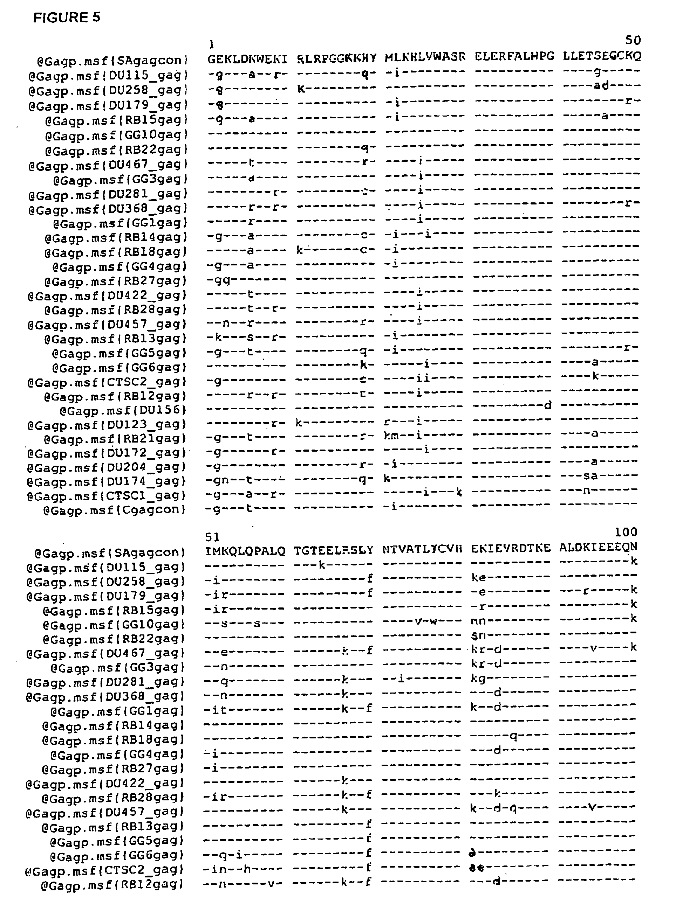

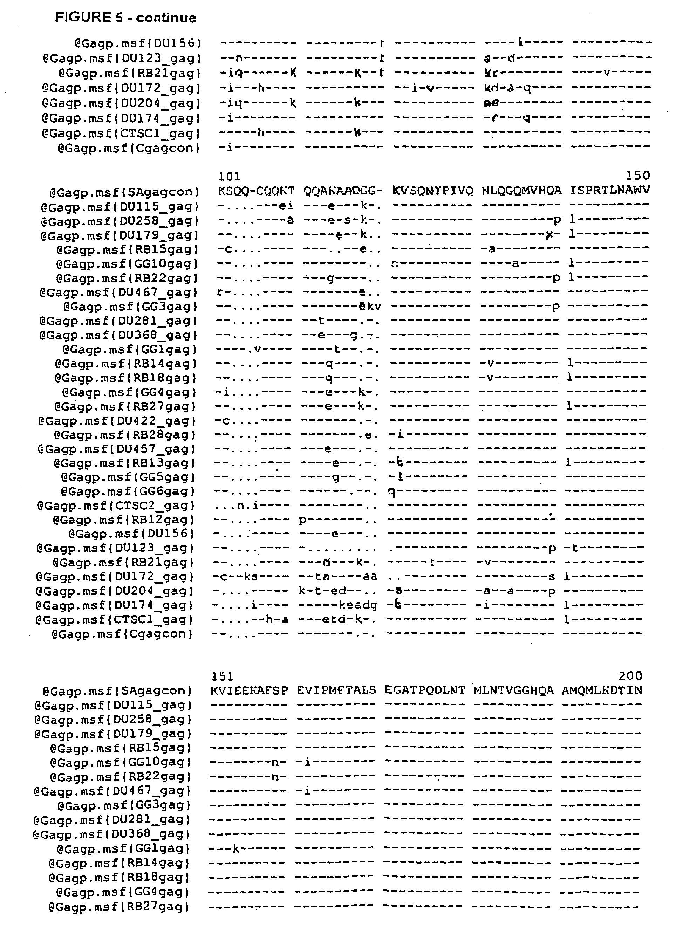

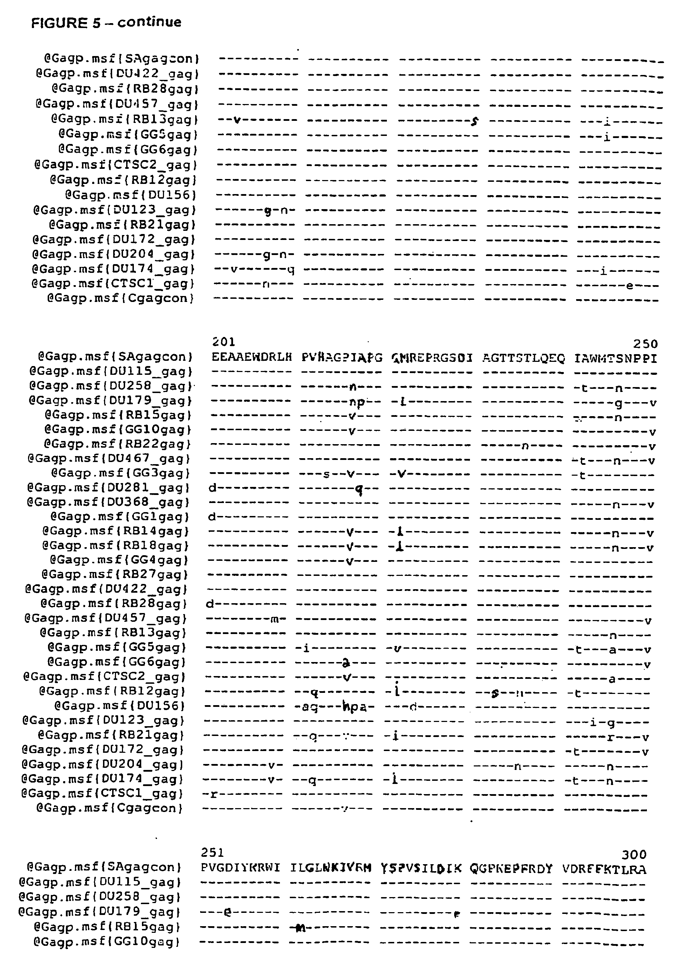

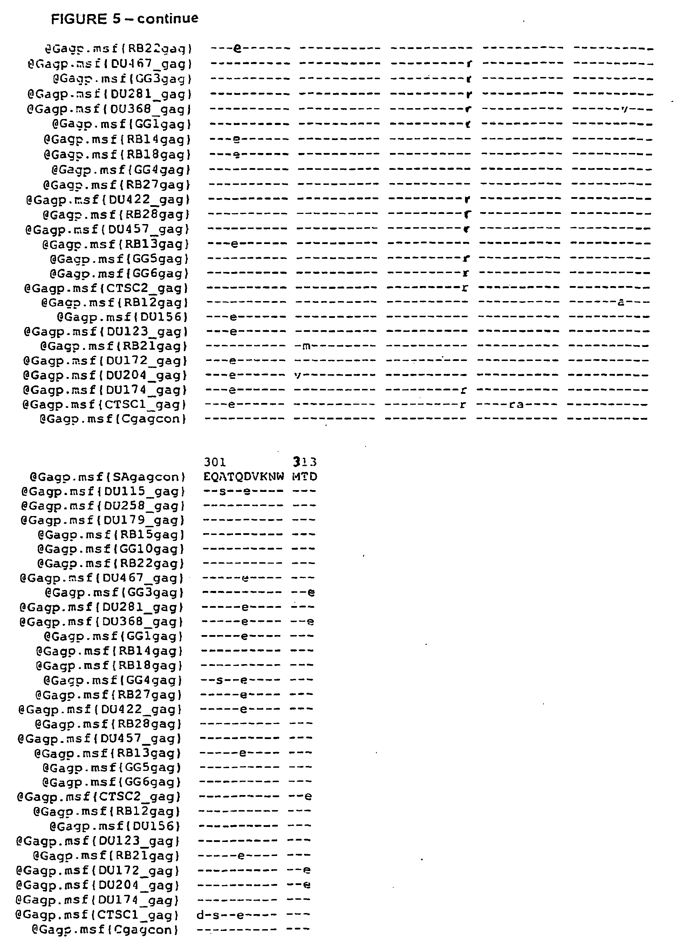

- **Figure 5**: shows how the sequences of the *gag* genes of each of a number of isolates varies from the South African consensus sequence for the *gag* gene which was developed according to the present invention;

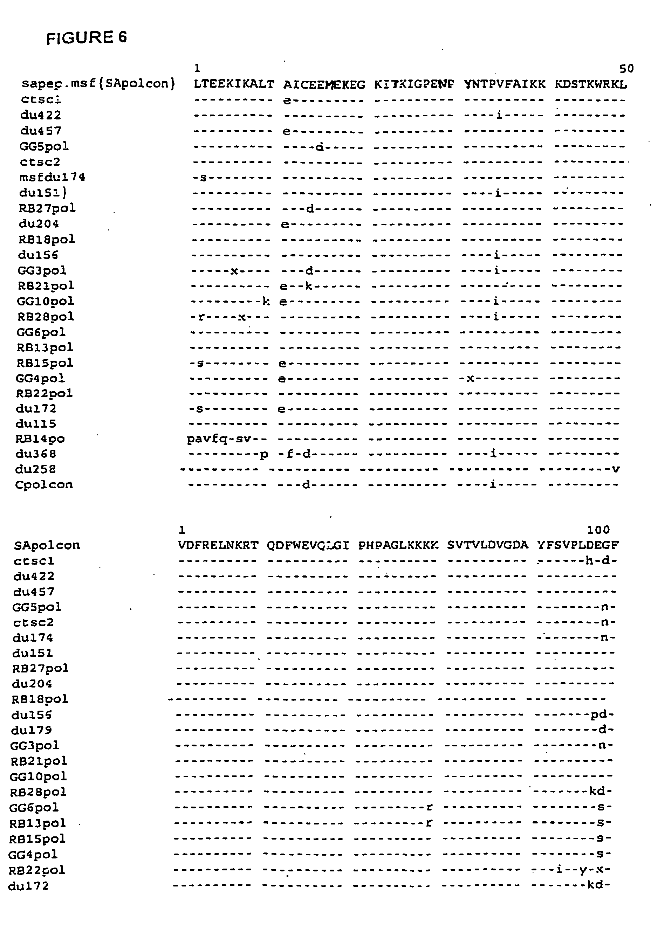

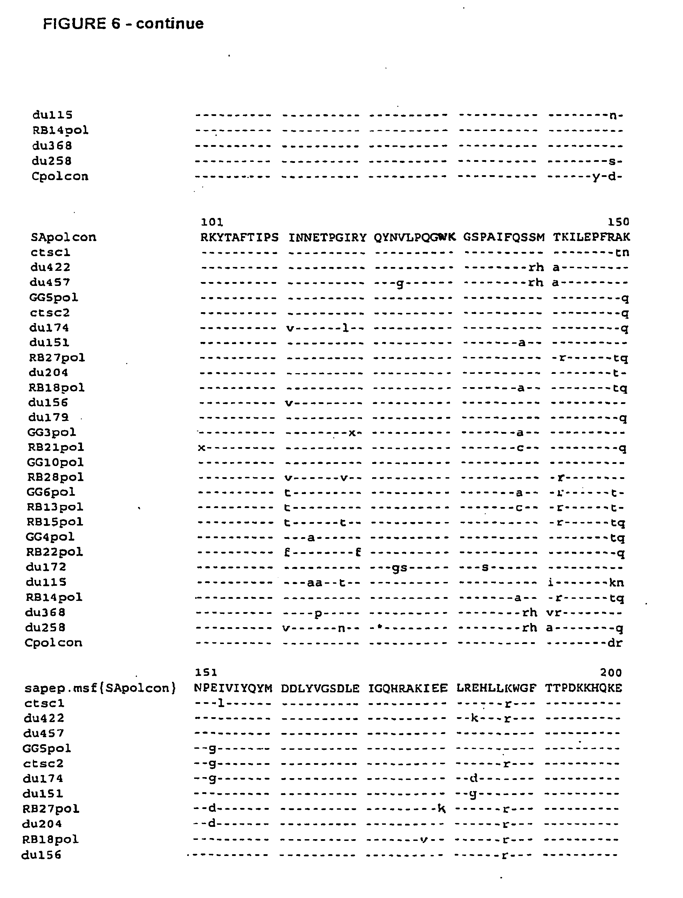

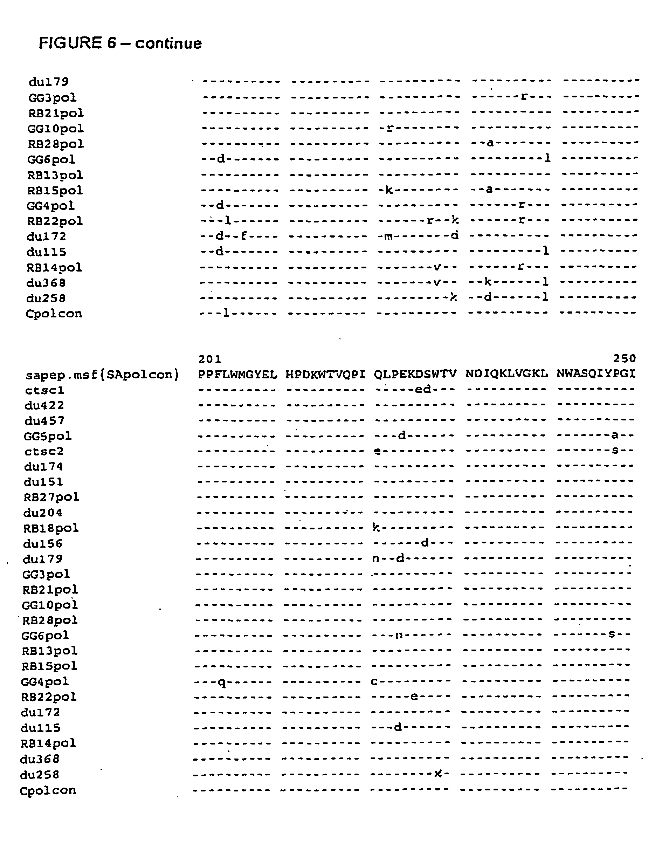

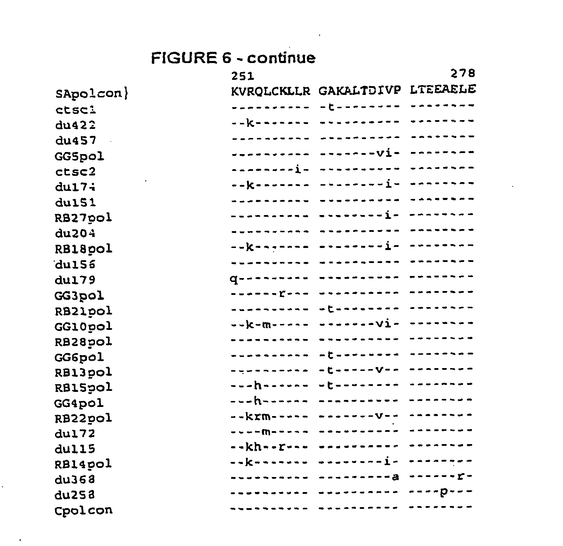

- **Figure 6**: shows how the sequences of the *pol* genes of each of a number of isolates varies from the South African consensus sequence for the *pol* gene which was developed according to the present invention;

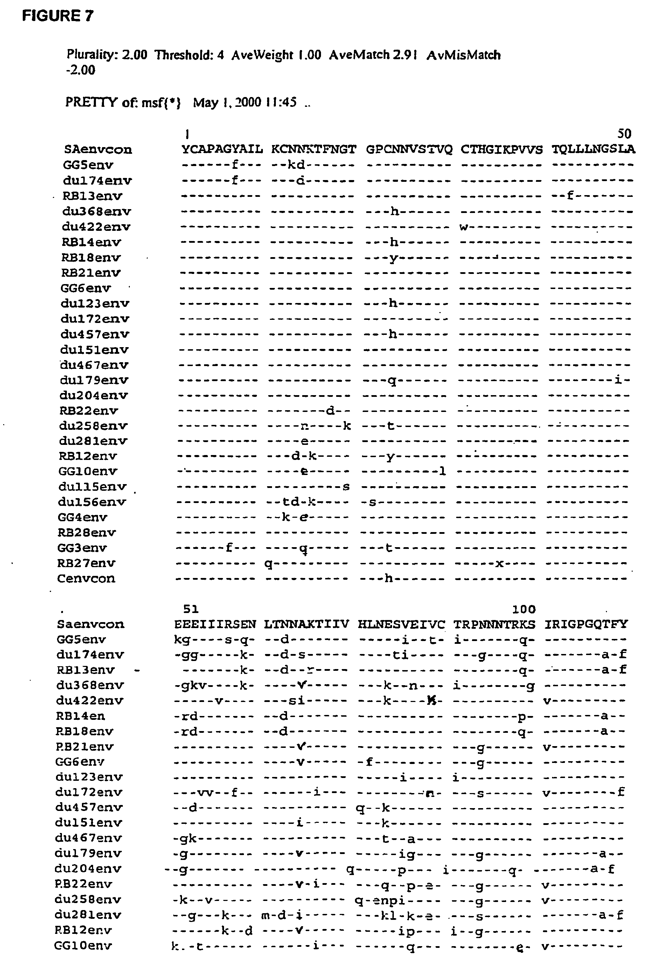

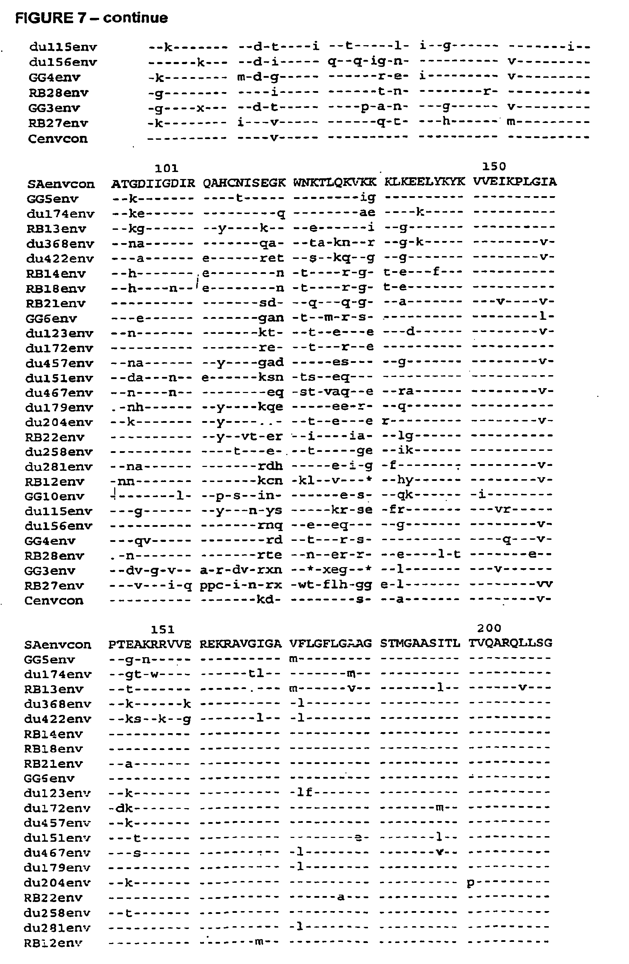

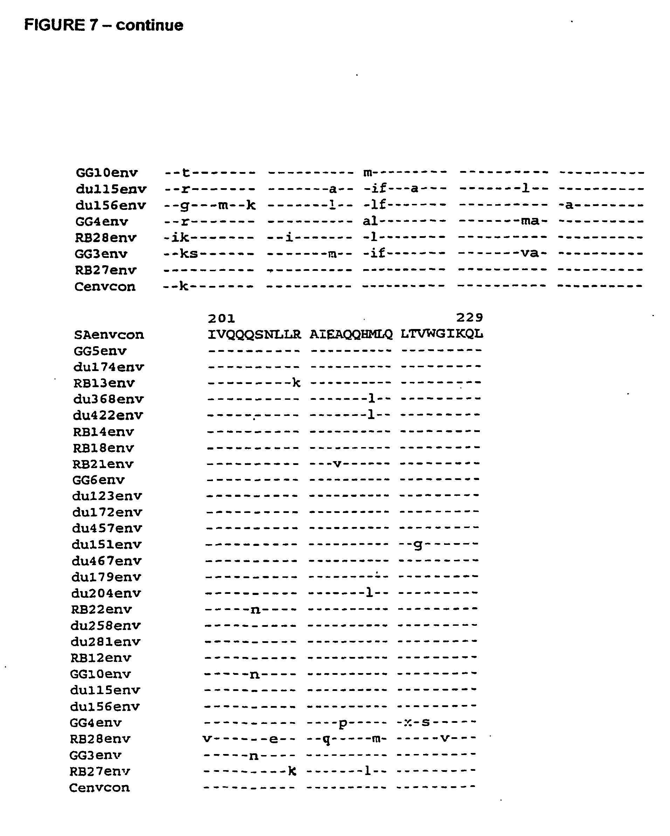

- **Figure 7**: shows how the sequences of the *env* genes of each of a number of isolates varies from the South African consensus sequence for the *env* gene which was developed according to the present invention;

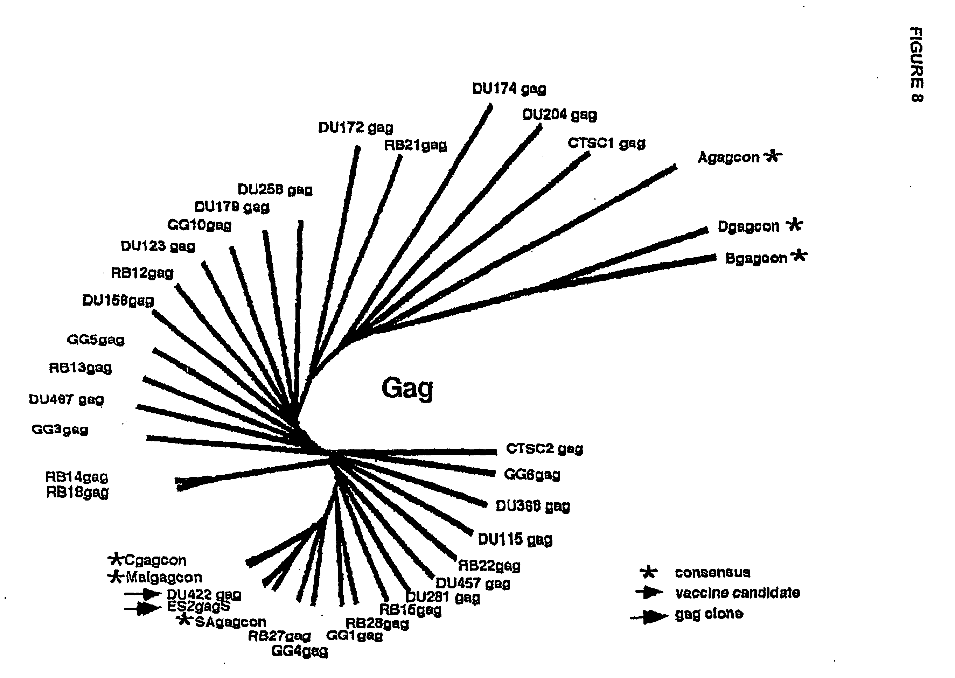

- **Figure 8**: shows a phylogenetic tree of amino acid sequences of various HIV- 1 subtype C isolates based on the sequences of the (partial) *gag* gene of the various isolates and includes a number of consensus sequences as well as the South African consensus sequence **disclosed herein** and a selected isolate, Du422.

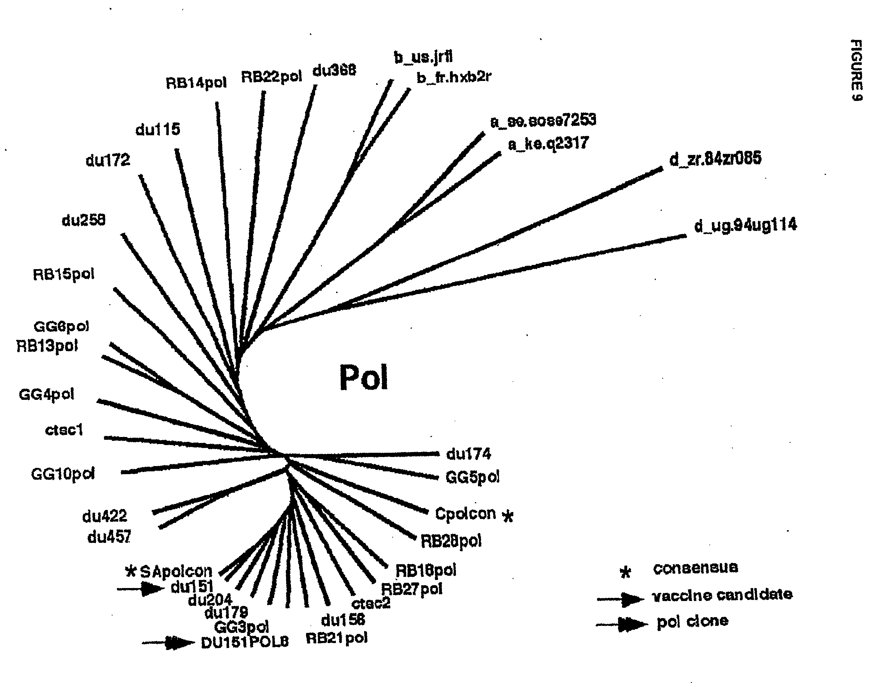

- **Figure 9**: shows a phylogenetic tree of amino acid sequences of various HIV- 1 subtype C isolates based on the sequences of the (partial) *pol* gene of the various isolates and includes a CpoI consensus sequence as well as a South African consensus sequence **disclosed herein** and a selected isolate, Du151.

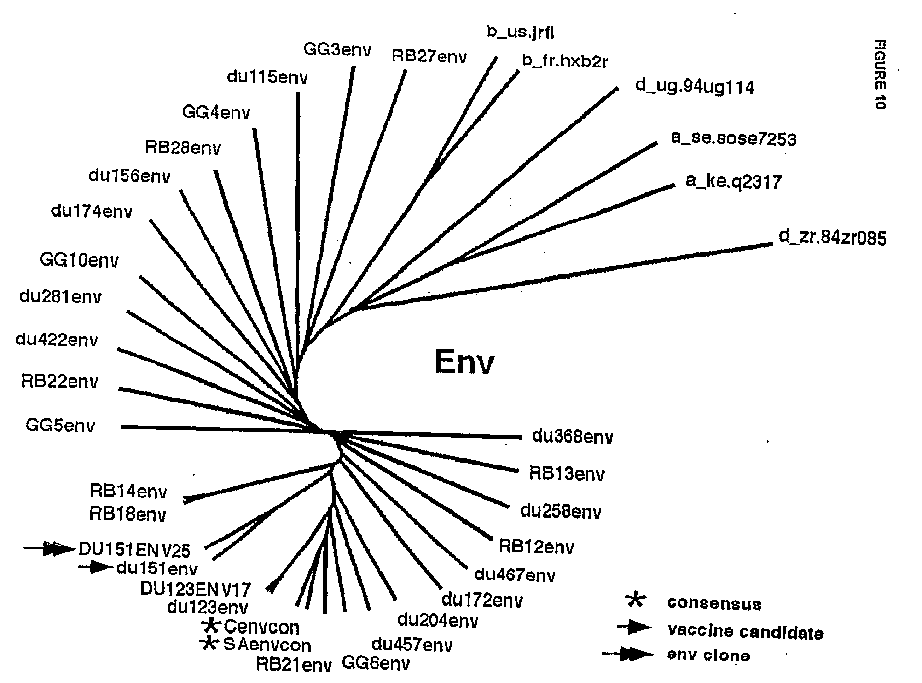

- **Figure 10**: shows a phylogenetic tree of amino acid sequences of various HIV- 1 subtype C isolates based on the sequences of the (partial) *env* gene of the various isolates and includes a Cenv consensus sequence as well as a South African consensus sequence **disclosed herein** and a selected isolate, Du151.

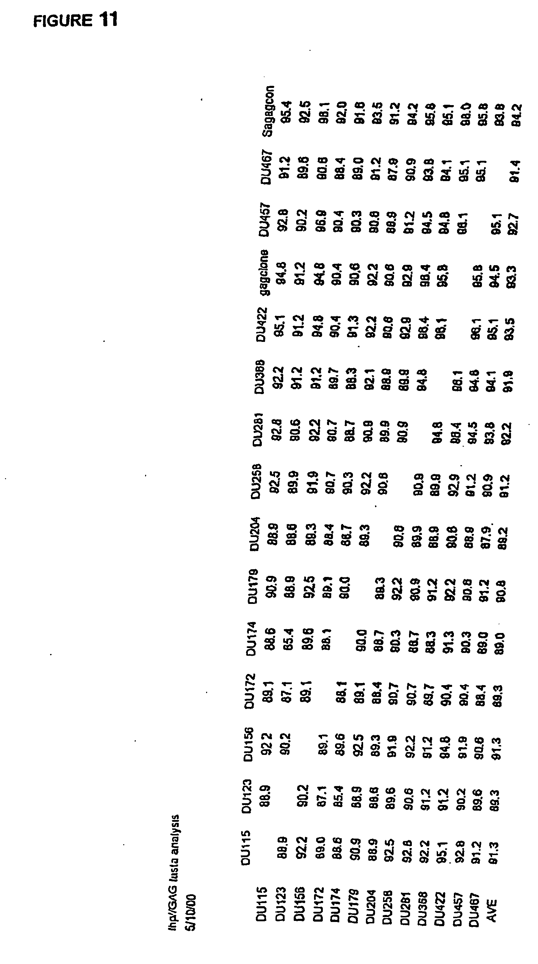

- **Figure 11**: shows the percentage amino acid sequence identity of the sequenced *gag* genes of the various isolates in relation to one another, to the *gag* clone and to the South African consensus sequence for the *gag* gene and is based on a pairwise comparison of the *gag* genes of the isolates;

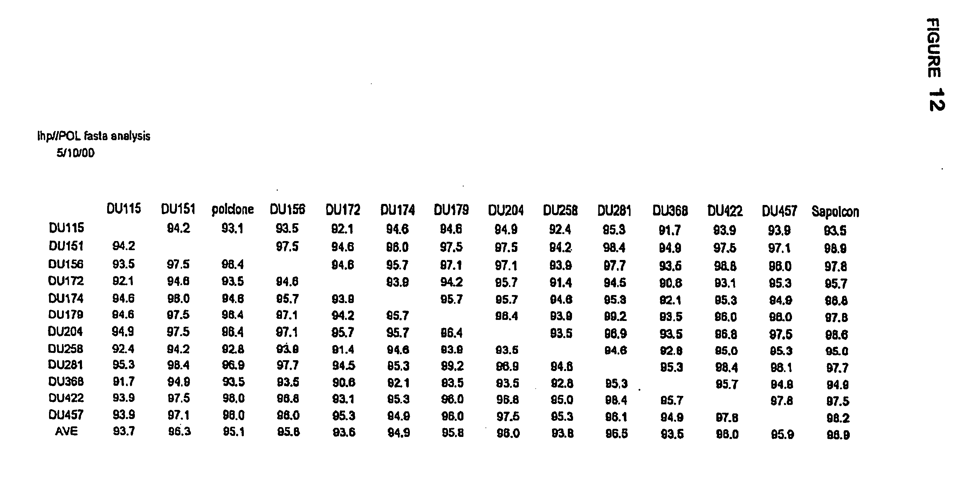

- **Figure 12**: shows the percentage amino acid sequence identity of the sequenced *pol* genes of the various isolates in relation to one another, to the *pol* clone and to the South African consensus sequence for the *pol* gene and is based on a pairwise comparison of the *pol* genes of the isolates;

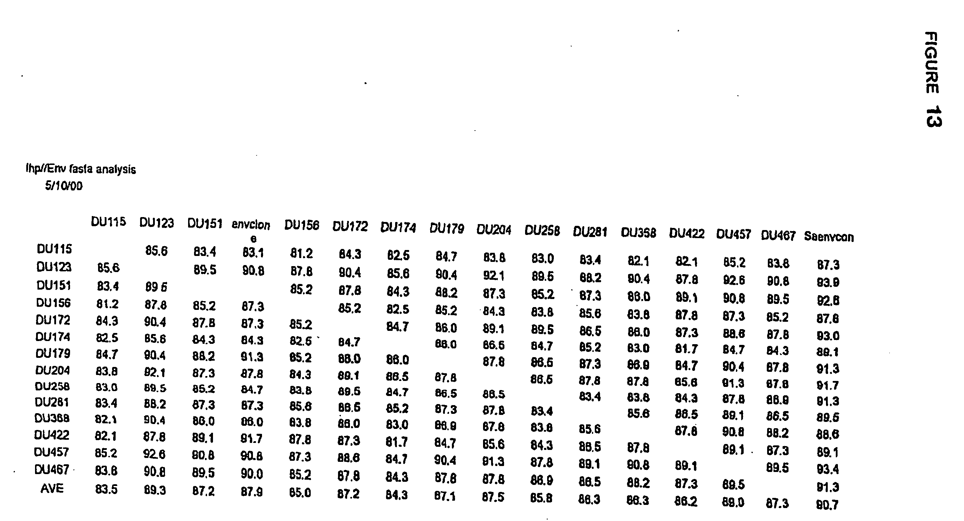

- **Figure 13**: shows the percentage amino acid sequence identity of the sequenced *env* genes of the various isolates in relation to one another, to the *env* clone and to the South African consensus sequence for the *env* gene and is based on a pairwise comparison of the *env* genes of the isolates;

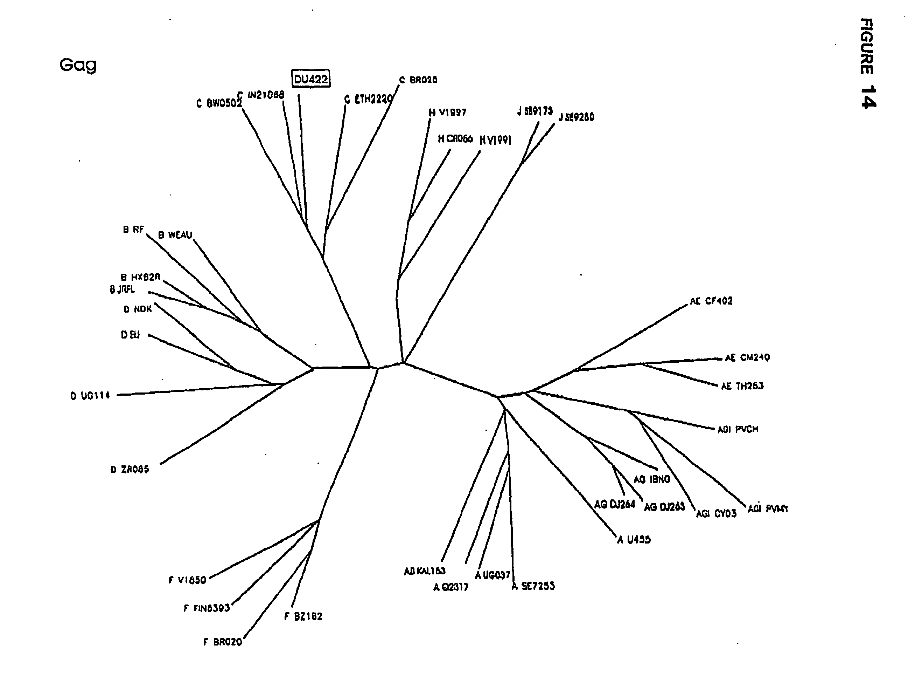

- **Figure 14**: shows a phylogenetic tree analysis of nucleic acid sequences of various HIV-1 subtype C isolates (or vaccine strains) based on the complete sequences of the gag genes of the various isolates and shows the gag gene from a selected isolate, Du 422, compared to the other subtype C sequences;

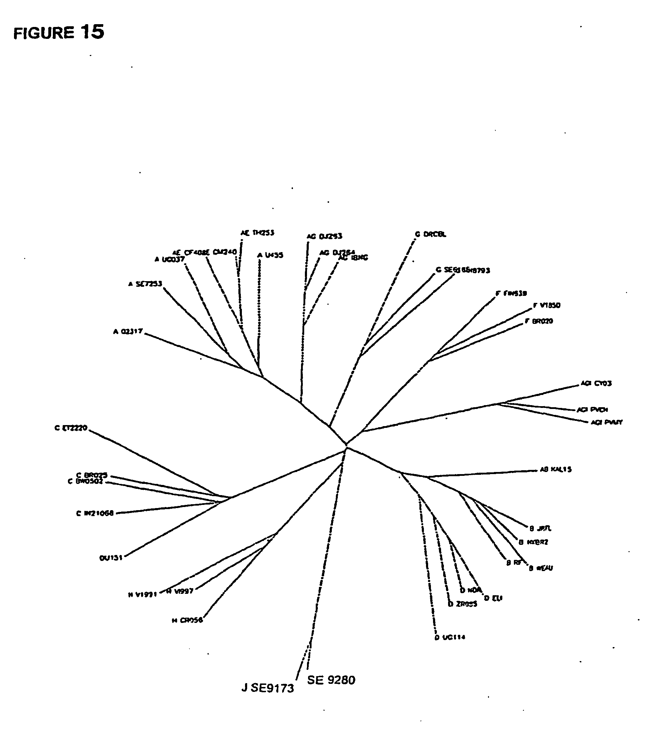

- **Figure 15**: shows a phylogenetic tree analysis of nucleic acid sequences of various HIV-1 subtype C isolates (or vaccine strains) based on the complete sequences of the *pol* genes of the various isolates and shows the *pol* gene from a selected isolate. Du151. compared to the other subtype C sequences;

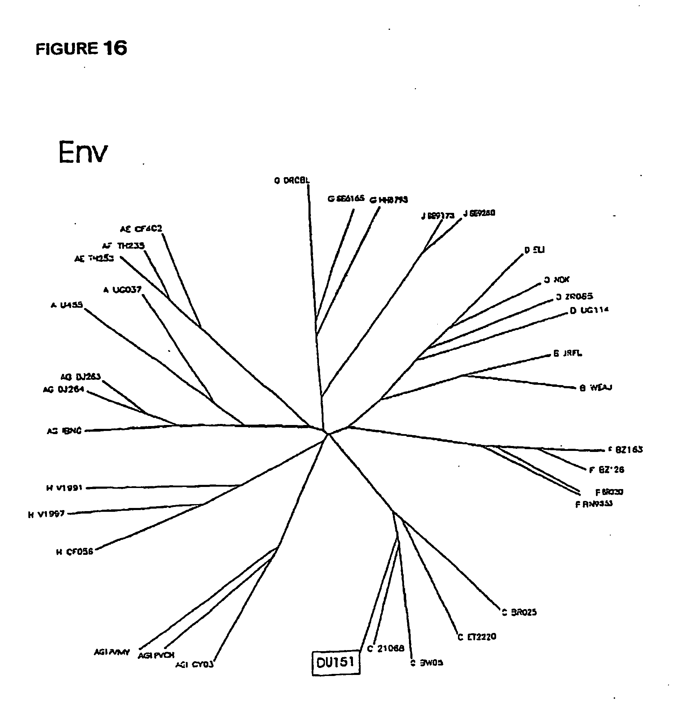

- **Figure 16**: shows a phylogenetic tree analysis of nucleic add sequences of various HIV-1 stubtype C isolates (or vaccine strains) based on the complete sequences of the *env* gene of the various isolates and shows the env gene from a selected isolate. Du151, compared to the other subtype C sequences: and

### LIST OF SEQUENCES

- Sequence ID No 1: shows the partial amino acid sequence of the sequenced *gag* gene of the isolate Du422, derived from the nucleic acid sequence (see Figure 5).
- Sequence ID No 2: shows the partial amino acid sequence of the sequenced *pol* gene of the isolate Du151, derived from the nucleic acid sequence (see Figure 6).
- Sequence ID No 3: shows the partial amino acid sequence of the sequenced *env* gene of the isolate Du151, derived from the nucleic acid sequence (see Figure 7).
- Sequence ID No 4: shows the nucleic acid sequence (DNA) sequence of the *gag* gene of the isolate Du422, resynthesized with modifications to reflect human codon usage for the purpose of increased expression.
- Sequence ID No 5: shows the amino acid sequence of the sequenced *gag* gene of the isolate Du422, derived from the codon-optimized nucleic acid.
- Sequence ID No 6: shows the nucleic acid sequence (DNA) sequence of the *pol* gene of the isolate Du151, resynthesized with modifications to reflect human codon usage for the purpose of increased expression.
- Sequence ID No 7: shows the amino acid sequence of the sequenced *pol* gene of the isolate Du151, derived from the codon-optimized nucleic acid.
- Sequence ID No 8: shows the nucleic acid sequence (DNA) sequence of the *env* gene of the isolate Du151, resynthesized with modifications to reflect human codon usage for the purpose of increased expression.
- Sequence ID No 9: shows the amino acid sequence of the sequenced *env* gene of the isolate Du151, derived from the codon-optimized nucleic acid.
- Sequence ID No 10: shows the nucleic acid sequence (cDNA) of the sequenced (not codon- optimized) *env* gene of the isolate Du179.
- Sequence ID No 11: shows the amino acid sequence of the sequenced (not codon-optimized) *env* gene of the isolate Du179.
- Sequence ID No 12: shows the consensus amino acid sequence for the partial *gag* gene of HIV-1 subtype C.
- Sequence ID No 13: shows the consensus amino acid sequence for the partial *pol* gene of HIV-1 subtype C.
- Sequence ID No 14: shows the consensus amino acid sequence for the partial *env* gene of HIV-1 subtype C.

### DETAILED DESCRIPTION OF THE INVENTION

This invention relates to the selection of HIV-1 subtype isolates and the use of their genes and modifications and derivatives thereof in making prophylactic and therapeutic pharmaceutical compositions and formulations, and in particular vaccines against HIV-1 subtype C. The compositions could therefore be used either prophylactically to prevent infection or therapeutically to prevent or modify disease. A number of factors must be taken into consideration in the development of an HIV vaccine and one aspect of the present invention relates to a process for the selection of suitable HIV isolates for the development of a vaccine.

The applicant envisages that the vaccine developed according to the above method could be used against one or more HIV subtypes other than HIV-1 subtype C.

An HIV vaccine aims to elicit both a CD8+ cytotoxic T lymphocyte (CTL) immune response as well as a neutralizing antibody response. Many current vaccine approaches have primarily focused on inducing a CTL response. It is thought that the CTL response may be more important as it is associated with the initial control of viral replication after infection, as well as control of replication during disease, and is inversely correlated with disease progression (Koup *et al*., 1994, Ogg *et al.,* 1999 Schmitz *et al.,* 1999). The importance of CTL in protecting individuals from infection is demonstrated by their presence in highly exposed seronegative individuals such as sex-workers (Rowland-Jones *et al.,* 1998).

Knowledge of genetic diversity is highly relevant to the design of vaccines aiming at eliciting a cytotoxic T-lymphocyte (CTL) response. There are many CTL epitopes in common between viruses, particularly in the *gag* and *pol* region of the genome (HIV Molecular Immunology Database, 1998). In addition, several studies have now shown that there is a cross-reactive CTL response: individuals vaccinated with a subtype B-based vaccine could lyse autologous targets infected with a diverse group of isolates (Ferrari *et al.,* 1997); and CTLs from non-B infected individuals could lyse subtype B-primed targets (Betts *et al*. 1997; Durali *et al*, 1998). A comparison of CTL epitopes in the HIV-1 sequence database shows about 40% of gp41 and 84% of p24 epitopes are identical or have only one amino acid difference between subtypes. Although this is a very crude analysis and does not take into consideration populations or dominant responses to certain epitopes, it does however indicate that there is a greater conservation of cytotoxic T epitopes within a subtype compared to between subtypes and that there will be a greater chance of a CTL response if the challenge virus is the same subtype as the vaccine strain.

The importance of genetic diversity in inducing a neutralizing antibody response appears to be less crucial. In general, neutralization serotypes are not related to genetic subtype. Some individuals elicit antibodies that can neutralize a broad range of viruses, including viruses of different subtypes while others fail to elicit effective neutralizing antibodies at all (Wyatt and Sodroski, 1998; Kostrikis *et al.,* 1996; Moore *et al.,* 1996). As neutralizing antibodies are largely evoked against functional domains of the virus which are essentially conserved, it is probable that HIV-1 genetic diversity may not be relevant in producing a vaccine designed to elicit neutralizing antibodies.

Viral strains used in the design of a vaccine need to be shown by genotypic analysis to be representative of the circulating strains and not an unusual or outlier strain. In addition, it is important that a vaccine strain also has the phenotype of a recently transmitted virus, which is NSI and uses the CCR5 co-receptor.

A process was developed to identify appropriate strains for use in developing a vaccine for HIV-1 subtype C. Viral isolates from acutely infected individuals were collected. They were sequenced in the *env, gag* and *pol* regions and the amino acid sequences for the *env, gag* and *pol* genes from these isolates were compared. A consensus sequence, the South African consensus sequence, was then formed by selecting the most frequently appearing amino acid at each position. The consensus sequence for each of the *gag, pol* and *env* genes of HIV-1 subtype C also forms an aspect of the invention. Appropriate strains for vaccine development were then selected from these isolates by comparing them with the consensus sequence and characterising them phenotypically.

In order to select for NSI strains which use the CCR5 co-receptor, a well established sex worker cohort was used to identify the appropriate strains. Appropriate strains were identified from acutely infected individuals by comparing them with the consensus sequence which had been formed. Viral isolates from fifteen acutely infected individuals were sequenced in the *env, gag* and *pol* and phenotypically characterised. These sequences were compared with viral isolates from fifteen asymptomatic individuals from another region having more than 500 CD4 cells and other published subtype C sequences located in the Los Alamos Database (http://ww.hiv-web.lanl.gov/).

Three potential vaccine strains, designated Du151, Du422 and Du179, were selected. Du 151 and Du 422 were selected based on amino acid homology to the consensus sequence in all three gene regions *env, gag* and *pol,* CCR5 tropism and ability to grow and replicate in tissue culture. Du 179 is a R5X4 virus and was selected because the patient in which this strain was found showed a high level of neutralising antibodies. The nucleotide and amino acid sequences of the three gene regions of the three isolates and modifications and derivatives thereof **can be used.**

The vaccines i will be formulated in a number of different ways using a variety of different vectors. They involve encapsulating RNA or transcribed DNA sequences from the viruses in a variety of different vectors. The vaccines will contain at least part of the *gag* gene from the Du422 isolate, and at least part of the *pol* and *env* genes from the Du151 isolate of the present invention and/or at least part of the *env* gene from the Du179 isolate of the present invention or derivatives or modifications thereof.

Genes for use in DNA vaccines have been resynthesized to reflect human codon usage. The *gag* Du422 gene was designed so that the myristylation site and inhibitory sequences were removed. Similarly resynthesized gp 160 (the complete *env* gene consisting of gp 120 and gp 41) and *pol* genes will be expressed by DNA vaccines. The gp160 gene sequence has also been changed as described above for the *gag* gene to reflect human codon usage and the rev responsive element removed. The protease, inactivated reverse transcriptase and start of the RNAse H genes from Du151 *pol* are optimised for increased expression and will be joined with *gag* at an inserted Bgl1 site. The *gag-pol* frameshift will be maintained to keep the natural balance of *gag* to *pol* protein expression.

Another vaccine will contain DNA transcribed from the RNA for the *gag* gene from the Du422 isolate and RNA from the *pol* and *env* genes from the Du151 isolate and/or RNA from the *env* gene from the Du179 isolate. These genes could also be expressed as oligomeric envelope glycoprotein complexes (Progenics, USA) as published in J Virol 2000 Jan;74(2):627-43 (Binley, J.L. et al.), the adeno associated virus (AAV) (Target Genetics) and the Venezuelan equine encephalitus virus (US patent application USSN 60/216,995, which is incorporated herein by reference).

### The isolation and selection of viral strains for the design of a vaccine

The following criteria were used to select appropriate strains for inclusion into HIV-1 vaccines for Southern Africa:
that the strains be genotypically representative of circulating strains;
that the strain not be an outlier strain;
that the strain be as close as possible to the consensus amino acid sequence developed according to the invention for the *env, gag* and *pol* genes of HIV-1 subtype C;
that the strain have an R5 phenotype, i.e. a phenotype associated with transmission for selection of the RNA or cDNA to be included for the *env* region; and
that the vaccine be able to be grown in tissue culture.

The following procedure was followed in the selection of viral strains for the design of a vaccine. A well-established sex worker cohort in Kwazulu Natal, South Africa was used to identify the appropriate strains for use in an HIV vaccine. Viral isolates from 15 acutely infected individuals were sequenced in *env, gag* and *pol* and were also isolated and phenotypically characterised. These sequences were compared with a similar collection from asymptomatic individuals from the Gauteng region in South Africa as well as other published subtype C sequences.

### Patients

Individuals with HIV infection were recruited from 4 regions in South Africa. Blood samples were obtained from recently infected sex workers from Kwazulu-Natal (n=13). Recent infection was defined as individuals who were previously seronegative and had became seropositive within the previous year. Samples were also collected from individuals attending out-patients clinics in Cape Town (n=2), women attending antenatal clinics in Johannesburg (n=7) and men attending a STD clinic on a gold mine outside Johannesburg (n=8). The latter 2 groups were clinically stable and were classified as asymptomatic infections. Blood samples were collected in EDTA and used to determine the CD4 T cell count and genetic analysis of the virus. In the case of recent infections a branched chain (bDNA) assay (Chiron) to measure plasma viral load was done, and the virus was isolated. HIV-1 serostatus was determined by ELISA. The results of the CD4 T cell counts and the viral loads on the sex workers were established and information on the clinical status as at date of seroconversion, CD4, and data on the co-receptor usage is set out in Table 1 below.

### Virus isolation

HIV was isolated from peripheral blood mononuclear cells (PBMC) using standard co-culture techniques with mitogen-activated donor PBMC. 2x10⁶ patient PBMC were co-cultured with 2x10⁶ donor PBMC in 12 well plates with 2 ml RPMI 1640 with 20% FCS, antibiotics and 5% IL-2 (Boehringer). Cultures were replenished twice weekly with fresh medium containing IL-2 and once with 5x10⁵ /ml donor PBMC. Virus growth was monitored weekly using a commercial p24 antigen assay (Coulter). Antigen positive cultures were expanded and cultured for a further 2 weeks to obtain 40 mls of virus containing supernatant which was stored at -70°C until use. The results of the isolation of the viruses from the commercial sex workers is also shown in Table 1 below.

### Viral phenotypes

Virus-containing supernatant was used to assess the biological phenotype of viral isolates on MT-2 and co-receptor transfected cell lines. For the MT-2 assay, 500 ul of supernatant was incubated with 5x10° MT-2 cells in PRMI plus 10% FCS and antibiotics. Cultures were monitored daily for syncitia formation over 6 days. U87.CD4 cell expressing either the CCR5 or CXCR4 co-receptor were grown in DMEM with 10% FCS, antibiotics, 500 ug/ml G418 and 1 ug/ml puromycin. GHOST cells expressing minor co-receptors were grown in DMEM with 10% FCS, 500 ug/ml G418, 1 ug/ml puromycin and 100 ug/ml hygromycin. Cell lines were passaged twice weekly by trypsination. Co-receptor assays were done in 12 well plates; 5x10⁴ cells were plated in each well and allowed to adhere overnight. The following day 500ul of virus containing supernatant was added and incubated overnight to allow viral attachment and infection and washed three times the following day. Cultures were monitored on days 4, 8 and 12 for syncitia formation and p24 antigen production. Cultures that showed evidence of syncitia and increasing concentrations of p24 antigen were considered positive for viral growth. The results of co-receptor usage of the viruses from the commercial sex workers is also shown in Table 1.

**TABLE 1-COHORT OF ACUTE INFECTIONS FOR SELECTION OF VACCINE CANDIDATES**

| **Sample ID** | **Sero date** | **Sample date** | **Duration of infection** | **CD4 count** | **Viral load** | **Co-culture p24 pos** | **MT-2 assay** | **Biotype** |
|---|---|---|---|---|---|---|---|---|
| Du115 | 15 May 98 | 20 May 99 | 1 year | 437* | 7,597* | - | No isolate | - |
| Du123 | 17 Aug 98 | 17 Nov 98 | 3 mon | 841 | 19.331 | d6 (50pg) | NSI | R5 |
| Du151 | 12 Oct 98 | 2 Nov 98 | 1.5 mon | 367 | >500.000 | d6 (>1ng) | NSI | R5 |
| Du156 | 16 Nov 98 | 17 Nov 98 | <1 mon | 404 | 22.122 | d6 (>1ng) | NSI | R5 |
| Du172 | 16 Oct 98 | 17 Nov 98 | 1 mon | 793 | 1.916 | d6 (<50pg) | NSI | R5 |
| Du174 | 6 Oct 97 | 25 May 99 | 19.5 mon | 634* | 9,454* | d14 (>Ing) | NSI | R5 |
| Du179 | 13 Aug 97 | 20 May 99 | 21 mon | 394* | 1,359* | d7 (<50pg) | SI | R5x4 |
| Du204 | 20 May 98 | 20 May 99 | 1 year | 633* | 8.734* | d7 (<50pg) | NSI | R5 |
| Du258 | 3 June 98 | 22 Jun 99 | 1 year | 433* | 9,114* | - | No isolate | - |
| Du281 | 24 July 98 | 17 Nov 98 | 4 mom | 594 | 24.689 | d6 (Ing) | NSI | R5 |
| Du285 | 2 Oct 98 | - | - | 560* | 161* | - | No isolate | - |
| Du368 | 8 Apr 98 | 24 Nov 98 | 7.5 mon | 670 | 13,993 | d6 (300pg) | NSI | R5 |
| Du422 | 2 Oct 98 | 28 Jan 99 | 4 mon | 397 | 17,118* | d6 (600 pg) | NSI | R5 |
| Du457 | 17 Aug 98 | 17 Nov 98 | 3 mon | 665 | 6,658 | - | No isolate | - |
| Du467 | 26 Aug 98 | - | - | 671 | 19.268 | - | No isolate | - |

| | | | | | | | | |
|---|---|---|---|---|---|---|---|---|
| * date from Nov 98 | | | | | | | | |

### Sequencing

RNA was isolated from plasma and the gene fragments were amplified from RNA using reverse transcriptase to generate a cDNA followed by PCR to generate amplified DNA segments. The positions of the PCR primers are as follows, with the second of each primer pair being used as the reverse transcriptase primer in the cDNA synthesis step (numbering using the HIV-1 HXBr sequence): *gag*1 (790-813, 1282-1303), *gag*2 (1232-1253 , 1797-1820), *pol*1 (2546-2573 , 3012-3041), *pol*2(2932-2957 , 3492-3515), *env*1 (6815-6838, 7322-7349), env2 (7626-7653 , 7963-7986). The amplified DNA fragments were purified using the QIAQUICK PCR Purification Kit (Qiagen, Germany). The DNA fragments were then sequenced using the upstream PCR primers as sequencing primers. Sequencing was done using the Sanger dideoxyterminator strategy with fluorescent dyes attached to the dideoxynucleotides. The sequence determination was made by electrophoresis using an ABI 377 Sequencer. A mapped illustration of an HIV-1 proviral genome showing the *pol, gag* and *env* regions sequenced as described above, is shown in Figure 1. The following regions were sequenced (numbering according to HXBr, Los Alamos database); 813 - 1282 (gag1); 1253 - 1797 (gag2); 2583 - 3012 (pol1); 2957-3515 (pol2); 6938 - 7322 (env1); 7653 - 7963 (env2), as illustrated in Figure 1.

### Genotypic characterisation

To select the vaccine isolate or isolates, a survey covering portions of the three major HIV genes *gag* (313 contiguous codons, 939 bases), *pol* (278 contiguous codons, 834 bases) and *env* (229 codons in two noncontigous segments, 687 bases) was done (Figure 1). The map of Figure 1 shows the 5'long terminal repeat, the structural and functional genes (*gag, pol* and *env*) as well as the regulatory and accessory proteins (*vif, tat, rev, nef, vpr* and *vpu*). The gag open reading frame illustrates the regions encoding p17 matrix protein and the p24 core protein and the p7 and p6 nuclearcapsid proteins. The pol open reading frame illustrates the protease (PR) p15, reverse transcriptase (RT) p66 and the Rnase H integrase p51. The env open reading frame indicates the region coding for gp 120 and the region coding for gp41.

Of a total of 31 isolates, 14 were from the Durban cohort (DU), 15 were from Johannesburg (GG and RB) and 2 from Cape Town (CT). Of these 30 were sequenced in the *gag* region, 26 in the *pol* region and 27 in the *env* region. The isolates that were sequenced are shown in Table 2.

**TABLE 2 - LIST OF ISOLATES AND THE REGIONS GENES SEQUENCEED**

| Isolate | *Gag* sequence | *Pol* sequence | *Env* sequence |
|---|---|---|---|
| CTSC1 | ✔ | ✔ | - |
| CTSC2 | ✔ | ✔ | - |
| DU115 | ✔ | ✔ | ✔ |
| DU123 | ✔ | - | ✔ |
| DU151 | - | ✔ | ✔ |
| DU156 | ✔ | ✔ | ✔ |
| DU172 | ✔ | ✔ | ✔ |
| DU174 | ✔ | ✔ | ✔ |
| DU 179 | ✔ | ✔ | ✔ |
| DU204 | ✔ | ✔ | ✔ |
| DU258 | ✔ | ✔ | ✔ |
| DU281 | ✔ | - | ✔ |
| DU368 | ✔ | ✔ | ✔ |
| DU422 | ✔ | ✔ | ✔ |
| DU457 | ✔ | ✔ | ✔ |
| DU467 | ✔ | - | ✔ |
| GG1 | ✔ | - | - |
| GG10 | ✔ | ✔ | ✔ |
| GG3 | ✔ | ✔ | ✔ |
| GG4 | ✔ | ✔ | ✔ |
| GG5 | ✔ | ✔ | ✔ |
| GG6 | ✔ | ✔ | ✔ |
| RB12 | ✔ | - | ✔ |
| RB13 | ✔ | ✔ | ✔ |
| RB14 | ✔ | ✔ | ✔ |
| RB15 | ✔ | ✔ | - |
| RB18 | ✔ | ✔ | ✔ |
| RB21 | ✔ | ✔ | ✔ |
| RB22 | ✔ | ✔ | ✔ |
| RB27 | ✔ | ✔ | ✔ |
| RB28 | ✔ | ✔ | ✔ |

The nucleic acid sequences from the Durban (DU) Johannesburg (GG, RB) and Cape Town (CT) cohorts were phylogenetically compared to all available published subtype C sequences (obtained from the Los Alamos HIV Sequence Database) including sequences from the other southern African countries and the overall subtype C consensus from the Los Alamos HIV sequence database. This comparison was done to ensure that the selected vaccine isolates were not phylogenetic outliers when compared to the Southern African sequences and the results of the comparison are shown in Figure 2, Figure 3 and Figure 4. Figures 2 to 4 illustrate that the sequences from Southern Africa are divergent and that the Indian sequences form a separate distinct cluster from these African sequences. The South African sequences are not unique and, in general, are as related to each other as they are to other sequences from Southern Africa. Overall this suggests Indian sequences are unique from Southern African subtype C sequences and that we do not have a clonal epidemic in South Africa, but rather South African viruses reflect the diversity of subtype C viruses in the Southern African region

### Determination of a consensus sequence

Amino acid sequences were derived from the sequences shown in Table 2 and were used to determine a South African consensus sequence. The most frequently appearing amino acid at each position was selected as the consensus amino acid at that position. In this way, the consensus sequence was determined along the linear length of each of the sequenced gene fragments (gag, *pol* and *env* gene fragments). The alignments were done using the Genetics Computer Group (GCG) programs (Pileup and Pretty), which generates a consensus sequence in this manner. These resulted in the consensus sequence for each gene region. The alignments of the amino acid sequences and the resulting consensus sequences are shown in Figures 5, 6 and 7.

The phylogenetic tree of amino acids showing a comparison of the South African sequences is set out in Figures 8, 9 and 10. The ES2 *gag* S, which is the sequence of the cloned Du422 *gag* gene, Du151 *pol* (clone number) 8, which is the sequence of the cloned Du151 *pol* gene, and Du151 *env* (clone number) 25, which is the sequence of the cloned Du151 *env* gene, are vaccine clones. It can be seen from Figures 8, 9 and 10 that they are the same as the original isolates. These phylogenetic trees compare the relationship between the HIV proteins. South African isolates were compared with subtype A, B, C and D consensus sequences as well as with the South African consensus (Sagagcon) derived from the South African sequences, a Malawian consensus (Malgagcon) derived from Malawian sequences and overall consensuses (Cgagcon, Cpolcon and Cenvcon) derived from all subtype C sequences on the Los Alamos database.

The final choice of which isolate or isolates to use was based on the similarity of the sequence of the *gag, env* and *pol* genes of a particular isolate to the South African consensus sequence which had been derived as set out above as well as the availability of an R5 isolate which had good replication kinetics as shown in Table 1.

### Selection of Vaccine Isolates

Based on the considerations and methodology set out above, three strains were selected from the acute infection cohort as the vaccine strains. The first strain is Du 422 for the *gag* gene, the second strain is Du151 for the *pol* and *env* genes and the third strain is Du179 which is a possible alternative for the *env* gene. These three strains were selected for the following reasons.
1. At the time the samples were obtained, Du151 had been infected for 6 weeks and had a CD4 count of 367 cells per ul of blood and a viral load above 500,000 copies per ml of plasma. Given the high viral load, and the recorded time from infection, it is probable that the individual was still in the initial stages of viraemia prior to control of HIV replication by the immune system.
2. At the time the samples were obtained, Du422 had been infected for 4 months with a CD4 count of 397 cells per ul of blood and a viral load of 17,118 copies per ml of plasma. In contrast to Du151, this individual had already brought viral replication under control to a certain extent.
3. At the time the samples were obtained, Du179 had been infected for 21 months with a CD4 count of 394 cells per ul of blood and a viral load of 1,359 copies per ml of plasma.

Based on the analysis of the phylogenetic tree shown in Figure 8 showing the relationship between full length gp120 sequence and other isolates, and the amino acid pairwise comparison shown in Figure 11, the Du422 *gag* sequence was shown to be most similar to the South African consensus sequence shown in Figures 2 and 5. It shared 98% amino acid sequence identity with the consensus sequence. In addition, the average pairwise distance, which is the percentage difference between the DNA sequences, between the DU422 *gag* sequence and the other sequences from the seroconverters was the highest of any sequence derived from this cohort, at 93.5%, and nearly as high as the average distance of the isolates to the SA consensus sequence (94.2%). The Du422 gag gene was cloned and the specific clone gave values very similar to the original isolate: having a pairwise identity value with the SA consensus of (98%) and nearly as high an average identity value with the other isolates as the DU422 isolate (93.3%). Thus, both the original DU422 isolate sequence and the generated clone had the highest pairwise percentage similarity to other isolates with the minimal values all being above 90%.

The *pol* sequences showed the highest values for the pairwise comparisons. Based on the analysis of the phylogenetic tree shown in Figure 9 and the pairwise identity score with the SA consensus (98.9%) shown in Figure 12, we chose the DU151 isolate as the source of the *pol* gene. Other contributing factors in this decision were that this is the same isolate that was chosen for the source of the *env* gene and that this was an isolate with excellent growth properties *in vitro.* The actual *pol* gene clone from the DU151 isolate was somewhat more divergent from the SA consensus sequence (97.8%), and had a smaller average identity score when compared to the other isolates (95.1%). However, we judged the small increase in distance from the consensus not to be significant in this otherwise well conserved HIV-1 gene and therefore chose the DU151 *pol* gene for further development. Only one of the recent seroconverter sequences was less than 93% identical with the DU151 *pol* gene segment.

The *env* gene showed the greatest sequence diversity. Based on the analysis of the phylogenetic tree shown in Figure 10, we chose the DU151 *env* gene. The DU151 *env* gene segment shows an average pairwise comparison score with the other isolates of 87.2%, with the clone being slightly higher (87.9%). The DU151 isolate gene segment has a pairwise identity score of 92.6% with the SA consensus while the DU151 clone is at 91.3%. Finally, all pairwise identity scores are above 83% with either the DU151 isolate sequence or the clone when compared to the other recent seroconverters, as shown in Figure 13. These pairwise scores make the DU151 sequence similar to the best scores in this sequence pool and combine these levels of similarity with an R5 virus with good cell culture replication kinetics.

The clones representing the full length gene for each of the above viral genes were generated by PCR. Viral DNA present in cells infected with the individual isolates were used for the *pol* and *env* clones, and DNA derived directly from plasma by RT-PCR was used for the *gag* clone. Total DNA was extracted from the infected cell pellets using the QIAGEN DNeasy Tissue Kit. This DNA was used in PCR reactions using the following primers (HXBR numbering, Los Alamos database) in a nested PCR amplification strategy:
*gag:* outer,623-640, and 2391-2408. inner, 789-810 and 2330-2350
*pol:* outer,2050 -2073, and 5119-5148. inner,2085 -2108, and 5068-5094.
*env:* outer, 6195-6218, and 8807-8830. inner, 6225-6245, and 8758-8795.

The PCR products were blunt-end cloned into pT7Blue using the Novagen pT7Blue Blunt Kit. The inserts were characterized by doing colony PCR to identify clones with gene inserts. The identity of the insert was confirmed by sequencing the insert on both strands and comparing this sequence to the original sequence.

### Modification of clones

Several modifications were introduced to the cloned genes, as shown in Figures 23 to 28. In order to increase levels of expression of proteins, the DNA sequence was resynthesized and the following modifications were made:
- the codon usage was changed to reflect human codon usage for increased expression; and
- the inhibitory and rev responsive elements were also removed.

The modifications to the *gag* gene sequence of Du422 are shown In Sequence I.D. numbers 4 and 5.

Also for the DNA, modified vaccinia ankara (MVA) and BCG vaccines, the *pol* gene was truncated so that only the protease, reverse transcriptase and RNAse H regions of the *pol* gene will be expressed. In addition, the active site amino acid motive YMDD has been mutated to YMAA so that the expressed reverse transcriptase will be catalytically inactive. The modifications to the *pol* gene of Du151 are shown in sequence I.D. numbers 6 and 7.

### Synthetic genes

The complete *gag* and *env* genes were resynthesized to optimise the codons for expression in human cells, also shown in Sequence I.D. numbers 6 to 9. During this process the inhibitory sequences (INS) and rev responsive elements (RRE) are removed which has reported to result in increased expression. The *gag* gene myristylation signal was mutated as described above and as shown In Sequence I.D. numbers 4 and 5.

The following material has been deposited with the European Collection of Cell Cultures, Centre for Applied Microbiology and Research, Salisbury, Wiltshire SP4 OJG, United Kingdom (ECACC).

### Deposits

| **Material** | **ECACC Deposit No.** | **Deposit Date** |
|---|---|---|
| HIV-1 Viral isolate Du151 | Accession Number 00072724 | 27 July 2000 |
| HIV-1 Viral isolate Du179 | Accession Number 00072725 | 27 July 2000 |
| HIV-1 Viral isolate Du422 | Provisional Accession Number 00072726 | 27 July 2000 |
| | Provisional Accession Number 01032114 | 22 March 2001 |

The deposit was made under the provisions of the Budapest Treaty on the International Recognition of the Deposit of Microorganisms for the Purpose of Patent Procedure and regulations thereunder (Budapest Treaty).

### REFERENCES

UNAIDS. AIDS epidemic update. December 1999.
   www.unaids.org/hivaidsinfo/documents.html
Binley JM, Sanders RW, Clas B, Schuelke N, Master A, Guo Y, Kajumo F, Anselma DJ, Maddon PJ, Olson WC, Moore JP., J Virol 2000 Jan;74(2):627-43
Bjorndal, A., Sonnerborg, A., Tscherning, C., Albert, J. & Fenyo, E. M. (1999). Phenotypic characteristics of human immunodeficiency virus type 1 subtype C isolates of Ethiopian.
Connor, R., Sheridan, K., Ceraldini, D., Choe, S. & Landau, N. (1997). Changes in co-receptor use correlates with disease progression in HIV-1-infected individuals. J Exp Med 185, 621-628.
Durali D, Morvan J, Letourneur F, Schmitt D, Guegan N, Dalod M, Saragosti S, Sicard D, Levy JP & Gomard E (1998). Cross-reactions between the cytotoxic T-lymphocyte responses of human immunodeficiency virus-infected African and European patients. J Virol 72:3547-53.
Ferrari G, Humphrey W, McElrath MJ, Excler JL, Duliege AM, Clements ML, Corey LC, Bolognesi DP & Weinhold KJ (1997). Clade B-based HIV-1 vaccines elicit cross-clade cytotoxic T lymphocyte reactivities in uninfected volunteers. Proc Natl Acad Sci U S A 18;94(4):1396-401.
HIV Molecular Immunology Database 1998: Korber B, Brander C, Koup R, Walker B, Haynes B, & Moore J, Eds. Theoretical Biology and Biophysics Group, Los Alamos National Laboratory, Los Alamos, NM.
Kostrikis, L.G., Cao, Y., Ngai, H., Moore, J.P. & Ho, D.D (1996). Quantitative analysis of serum neutralization of human immunodeficiency virus type 1 from subtypes A, B, C, D, E, F, and I: lack of direct correlation between neutralization serotypes and genetic subtypes and evidence for prevalent serum-dependent infectivity enhancement. J. Virol. 70, 445-458.
Koup RA, Safrit JT, Cao Y, Andrews CA, McLeod G, Borkowsky W, Farthing C, Ho DD (1994). Temporal association of cellular immune responses with the initial control of viremia in primary human immunodeficiency virus type 1 syndrome. J Virol. 68(7):4650-5.
Moore JP, Cao Y, Leu J, Qin L, Korber B & Ho DD (1996). Inter- and intraclade neutralization of human imunodeficiency virus type 1: genetic clades do not correspond to neutralization serotypes but partially correspond to gp120 antigenic serotypes. J. Virol. 70, 427-444.
Ogg GS, Kostense S, Klein MR, Jurriaans S, Hamann D, McMichael AJ & Miedema F (1999). Longitudinal phenotypic analysis of human immunodeficiency virus type 1-specific cytotoxic T lymphocytes: correlation with disease progression. J Virol; 73(11):9153-60.
Peeters, M., Vincent, R., Perret, J.-L., Lasky, M., Patrel, D., Liegeois, F., Courgnaud, V., Seng, R., Matton, T., Molinier, S. & Delaporte, E. (1999). Evidence for differences in MT2 cell tropism according to genetic subtypes of HIV-1: syncitium-inducing variants seem rare among subtype C HIV-1 viruses. J Acquir Imm Def Synd 20, 115-121.
Richman, D. & Bozzette, S. (1994). The impact of the syncytium-inducing phenotype of human immunodeficiency virus on disease progression. J Inf Dis 169, 968-974.
Robertson DL, Anderson JP, Bradac JA, Carr JK, Foley B, Funkhouser RK, Gao R, Hahn BH, Kalish ML, Kuiken C, Learn GH Leitner T, McCutchan F, Osmanov S, Peeters M, Pieniazek D, Salminen M, Sharp PM, Wolinsky S, Korber B (2000). HIV nomenclature proposal. Science 7;288 (5463):55-6.
Rowland-Jones SL, Dong T, Fowke KR, Kimani J, Krausa P, Newell H, Blanchard T, Ariyoshi K, Oyugi J, Ngugi E, Bwayo J, MacDonald KS, McMichael AJ & Plummer FA (1998). Cytotoxic T-cell responses to multiple conserved epitopes in HIV-resistant prostitutes in Nairobi. J. Clin. Invest. 102 (9): 1758-1765.
Scarlatti, G., Tresoldi, E., Bjorndal, A., Fredriksson, R., Colognesi, C., Deng, H., Malnati, M., Plebani, A., Siccardi, A., Littman, D., Fenyo, E. & Lusso, P. (1997). In vivo evolution of HIV-1 co-receptor usage and sensitivity to chemokine-mediated suppression. Nat Med 3, 1259-1265.
Schmitz JE, Kuroda MJ, Santra S, Sasseville VG, Simon MA, Lifton MA, Racz P, Tenner-Racz K, Dalesandro M, Scallon BJ, Ghrayeb J, Forman MA, Montefiori DC, Rieber EP, Letvin NL, Reimann KA (1999). Control of viremia in simian immunodeficiency virus infection by CD8+ lymphocytes. Science 5;283(5403):857-60.
Summary Report: National HIV sero-prevalence survey of women attending public antenatal clinics in South Africa, 1999 (2000). Department of Health, Directorate: Health Systems Research & Epidemiology, April 2000.
Tscherning, C., Alaeus, A., Fredriksson, R., Bjorndal, A., Deng, H., Littman, D., Fenyo, E. M. & Alberts, J. (1998). Differences in chemokine co-receptor usage between genetic subtypes of HIV-1. Virology 241, 181-188.
Wyatt R and Sodroski J (1998). The HIV-1 envelope glycoproteins: Fusogens, antigens and immunogens. Science, 280 (5371):1884-8.
Wyatt R, Kwong, Desjardins E, Sweet RW, Robinson J, Hendrickson WA & Sodroski JG (1998). The antigenic structure of the HIV gp120 envelope glycoprotein. Nature, 393(6686):705-11.

### SEQUENCE LISTING

Sequence I.D. No: 1: 
GEKLDTWEKI RLRPGGKKHY MLKHIVWASR ELERFALNPG LLETSEGCKQ IMKQLQPALQ 60
TGTEELKSLY NTVATLYCVH EKIEVRDTKE ALDKIEEEQN KCQQKTQQAK AADGKVSQNY 120
PIVQNLQGQM VHQAISPRTL NAWVKVIEEK AFSPEVIPMF TALSEGATPQ DLNTMLNTVG 180
GHQAAMQMLK DTINEEAAEW DRVHPVHAGP IAPGQMREPR GSDIAGTTST LQEQIAWMTS 240
NPPIPVGDIY KRWIILGLNK IVRMYSPVSI LDIRQGPKEP FRDYVDRFFK TLRAEQATQE 300
VKNWMTD
Sequence I.D. No: 2: 
PLTEEKIKAL TAICEEMEKE GKITKIGPEN PYNTPIFAIK KKDSTKWRKL VDFRELNKRT 60
QDFWEVQLGI PHPAGLKKKK SVTVLDVGDA YFSVPLDEGF RKYTAFTIPS INNETPGIRY 120
QYNVLPQGWK GSPAIFQGSM TKILEPFRAQ NPEIVIYQYM DDLYVGSDLE IGQHRAKIEE 180
LREHLLKWGF TTPDKKHQKE PPFLWMGYEL HPDKWTVQPI QLPEKDSWTV NDIQKLVGKL 240
NWASQIYPGI KVRQLCKLLR GTKALTDIVP LTEEAELE 278
Sequence I.D. No: 3: 
YCAPAGYAIL KCNNKTFNGT GPCNNVSTVQ CTHGIMPVVS TQLLLNGSLA EEEIIIRSEN 60
LTNNIKTIIV HLNKSVEIVC TRPNNNTRKS IRIGPGQTFY ATGEIIGNIR EAHCNISKSN 120
WTSTLEQVKK KLKEHYNKTI EFNPPSGGDL EVTTHSFNCR GEFFYCNTTK LFSNNSDSNN 180
ETITLPCKIK QIINMWQKVG RAMYAPPIEG NITCKSNITG LLLTRDGGKN TTNEIFRPGG 240
GNMKDNWRSE LYKYKVVEIE PLGVAPTKSK RRVVEREKRA VGLGAVLLGF LGAAGSTMGA 300
ASITLTVQAR QLLSGIVQQQ SNLLRAIEAQ QHMLQ 335
Sequence I.D. No: 4: Du422 synthesised *gag* gene 
1
ATGGCTGCTCGCGCATCTATCCTCAGAGGC
121 GAAAAGTTGGATAAGTGGGAAAAAATCAGACTCAGGCCAGGAGGTAAAAAACACTACATG
181 CTGAAGCATATCGTGTGGCATCTAGGGAGTTGGAGAGATTTGCACTGAACCCCGGACTG
241 CTGGAAACCTCAGAGGGCTGTAAGCAAATCATGAAACAGCTCCAACCAGCCTTGCAGACC
301 GGAACAGAAGAGCTGAAGTCCCTTTACAATACCGTGGCAACCCTCTATTGCGTCCACGAG
361 AAGATCGAGGTGAGAGACACAAAGGAGGCCCTGGACAAAATCGAGGAGGAGCAGAATAAG
421 TGCCAGCAGAAGACCCAGCAGGCAAAGGCTGCTGACGGAAAGGTCTCTCAGAACTATCCT
481 ATCGTTCAGAACCTTCAGGGGCAGATGGTGCACCAAGCAATCAGCCCTAGAACCCTGAAC
541 GCATGGGTGAAGGTGATCGAGGAGAAAGCCTTTTCTCCCGAGGTTATCCCCATGTTTACC
601 GCCCTGAGCGAAGGCGCCACTCCTCAAGACCTGAACACTATGCTGAACACAGTGGGAGGA
661 CACCAGGCCGCTATGCAGATGTTGAAGGATACCATCAACGAGGAGGCAGCCGAATGGGAC
721 CGCCTCCACCCCGTGCACGCCGGACCTATCGCCCCCGGACAAATGAGAGAACCTCGCGGA
781 AGTGATATTGCCGGTACTACCAGCACCCTTCAAGAGCAGATTGCTTGGATGACCAGCAAC
841 CCACCCATCCCAGTGGGCGATATTTACAAAAGGTGGATTATTCTGGGGCTGAACAAAATT
901 GTGAGAATGTACTCCCCCGTCTCCATCCTCGACATCCGCCAAGGACCCAAGGAGCCTTTT
961 AGGGATTACGTGGACAGATTCTTCAAAACCCTTAGAGCTGAGCAAGCCACTCAGGAGGTT
1021 AAGAACTGGATGACAGATACTCTGCTCGTGCAAAACGCTAACCCCGATTGCAAAACCATC
1081 TTGAGAGCTCTCGGTCCAGGTGCCACCCTTGAGGAAATGATGACAGCATGTCAAGGCGTG
1141 GGAGGACCTGGGCACAAGGCCAGAGTTCTCGCTGAGGCCATGAGCCAGACAAACTCAGGC
1201 AATATCATGATGCAGAGGAGTAACTTTAAGGGTCCCAGGAGAATCGTCAAGTGCTTCAAT
1261 TGTGGCAAGGAGGGTCACATTGCCAGGAACTGCCGCGCCCCCAGGAAGAAAGGCTGCTGG
1321 AAGTGTGGCAAAGAGGGCCACCAGATGAAGGATTGCACCGAGCGCCAAGCAAACTTCCTG
 
1381 GGAAAGATTTGGCCCAGTCATAAGGGCCGCCCTGGCAACTTCCTTCAAAACAGACCCGAG
1441 CCTACCGCCCCCCCCGCTGAGTCTTTCAGATTTGAGGAGACCACCCCCGCTCCAAAGCAG
1501 GAGCCAATTGAGAGAGAGCCTCTCACCAGTCTCAAAAGCCTCTTTGGTAGCGACCCCCTC
1561 AGCCAATAA
Sequence I.D. No: 5: Du422 synthesised Gag Protein 
1 'ATGGCTGCTCGCGCATCTATCCTCAGAGGC
M A A R A S I L R G

121 GAAAAGTTGGATAAGTGGGAAAAAATCAGACTCAGGCCAGGAGGTAAAAAACACTACATG
41 E K L D K W E K I R L R P G G K K H Y M

181 CTGAAGCATATCGTGTGGGCATCTAGGGAGTTGGAGAGATTTGCACTGAACCCCGGACTG
61 L K H I V W A S R E L E R F A L N P G L

241 CTGGAAACCTCAGAGGGCTGTAAGCAAATCATGAAACAGCTCCAACCAGCCTTGCAGACC
81 L E T S E G C K Q I M K Q L Q P A L Q T

301 GGAACAGAAGAGCTGAAGTCCCTTTACAATACCGTGGCAACCCTCTATTGCGTCCACGAG
101 G T E E L K S L Y N T V A T L Y C V H E

361 AAGATCGAGGTGAGAGACACAAAGGAGGCCCTGGACAAAATCGAGGAGGAGCAGAATAAG
121 K I E V R D T K E A L D K I E E E Q N K

421 TGCCAGCAGAAGACCCAGCAGGCAAAGGCTGCTGACGGAAAGGTCTCTCAGAACTATCCT
141 C Q Q K T Q Q A K A A D G K V S Q N Y P

481 ATCGTTCAGAACCTTCAGGGGCAGATGGTGCACCAAGCAATCAGCCCTAGAACCCTGAAC
161 I V Q N L Q G Q M V H Q A I S P R T L N

541 GCATGGGTGAAGGTGATCGAGGAGAAAGCCTTTTCTCCCGAGGTTATCCCCATGTTTACC
181 A W V K V I E E K A F S P E V I P M F T

601 GCCCTGAGCGAAGGCGCCACTCCTCAAGACCTGAACACTATGCTGAACACAGTGGGAGGA
201 A L S E G A T P Q D L N T M L N T V G G

661 CACCAGGCCGCTATGCAGATGTTGAAGGATACCATCAACGAGGAGGCAGCCGAATGGGAC
221 H Q A A M Q M L K D T I N E E A A E W D

721 CGCCTCCACCCCGTGCACGCCGGACCTATCGCCCCCGGACAAATGAGAGAACCTCGCGGA
241 R L H P V H A G P I A P G Q M R E P R G

781 AGTGATATTGCCGGTACTACCAGCACCCTTCAAGAGCAGATTGCTTGGATGACCAGCAAC
261 S D I A G T T S T L Q E Q I A W M T S N

841 CCACCCATCCCAGTGGGCGATATTTACAAAAGGTGGATTATTCTGGGGCTGAACAAAATT
281 P P I P V G D I Y K R W I I L G L N K I

901 GTGAGAATGTACTCCCCCGTCTCCATCCTCGACATCCGCCAAGGACCCAAGGAGCCTTTT
301 V R M Y S P V S I L D I R Q G P K E P F

961 AGGGATTACGTGGACAGATTCTTCAAAACCCTTAGAGCTGAGCAAGCCACTCAGGAGGTT
321 R D Y V D R F F K T L R A E Q A T Q E V
 
1021        AAGAACTGGATGACAGATACTCTGCTCGTGCAAAACGCTAACCCCGATTGCAAAACCATC
341         K  N  W  M  T  D  T  L  L  V  Q  N  A  N  P  D  C  K  T  I

1081        TTGAGAGCTCTCGGTCCAGGTGCCACCCTTGAGGAAATGATGACAGCATGTCAAGGCGTG
361         L  R  A  L  G  P  G  A  T  L  E  E  M  M  T  A  C  Q  G  V

1141        GGAGGACCTGGCCACAAGGCCAGAGTTCTCGCTGAGGCCATGAGCCAGACAAACTCAGGC
381         G  G  P  G  H  K  A  R  V  L  A  E  A  M  S  Q  T  N  S  G

1201        AATATCATGATGCAGAGGAGTAACTTTAAGGGTCCCAGGAGAATCGTCAAGTGCTTCAAT
401         N  I  M  M  Q  R  S  N  F  K  G  P  R  R  I  V  K  C  F  N

1261        TGTGGCAAGGAGGGTCACATTGCCAGGAACTGCCGCGCCCCCAGGAAGAAAGGCTGCTGG
421         C  G  K  E  G  H  I  A  R  N  C  R  A  P  R  K  K  G  C  W

1321        AAGTGTGGCAAAGAGGGCCACCAGATGAAGGATTGCACCGAGCGCCAAGCAAACTTCCTG
441         K  C  G  K  E  G  H  Q  M  K  D  C  T  E  R  Q  A  N  F  L

1381        GGAAAGATTTGGCCCAGTCATAAGGGCCGCCCTGGCAACTTCCTTCAAAACAGACCCGAG
461         G  K  I  W  P  S  H  K  G  R  P  G  N  F  L  Q  N  R  P  E

1441        CCTACCGCCCCCCCCGCTGAGTCTTTCAGATTTGAGGAGACCACCCCCGCTCCAAAGCAG
481         P  T  A  P  P  A  E  S  F  R  F  E  E  T  T  P  A  P  K  Q

1501        GAGCCAATTGAGAGAGAGCCTCTCACCAGTCTCAAAAGCCTCTTTGGTAGCGACCCCCTC
501         E  P  I  E  R  E  P  L  T  S  L  K  S  L  F  G  S  D  P  L

1561        AGCCAA
521         S  Q
Sequence I.D. No: 6: Du151 synthesised *pol* gene 
451 AATTTCCT TCAGAACAGA CCAGAGCCAA
AAGGGTGTTC CCGGCCGGTC CGTTAAAGGA AGTCTTGTCT GGTCTCGGTT
501 CAGCCCCACC AGCAGAGAGC TTCAGGTTCG AAGAGACAAC CCCCGCTCCG
GTCGGGGTGG TCGTCTCTCG AAGTCCAAGC TTCTCTGTTG GGGGCGAGGC
551 AAACAGGAGC CGAGAGAAAG GGAACCCTTA ACTTCCCTCA AATCACTCTT
TTTGTCCTCG GCTCTCTTTC CCTTGGGAAT TGAAGGGAGT TTAGTGAGAA
601 TGGCAGCGAC CCCTTGTCTC AATAAAAATC GGCGGCCAGA CCCGGAGGC
ACCGTCGCTG GGGAACAGAG TTATTTTTAG CCGCCGGTCT GGGCCCTCCG
651 CCTGCTGGAC ACCGGCGCCG ACGACACCGT GCTGGAGGAC ATCAACCTGC
GGACGACCTG TGGCCGCGGC TGCTGTGGCA CGACCTCCTG TAGTTGGACG
701 CCGGCAAGTG GAAGCCCAAG ATGATCGGCG GCATCGGCGG CTTCATCAAG
GGCCGTTCAC CTTCGGGTTC TACTAGCCGC CGTAGCCGCC GAAGTAGTTC
751 GTGCGGCAGT ACGACCAGAT CCTGATCGAG ATCTGCGGCA AGAAGGCCAT
CACGCCGTCA TGCTGGTCTA GGACTAGCTC TAGACGCCGT TCTTCCGGTA
801 CGGCACCGTG CTGGTGGGCC CCACCCCCGT GAACATCATC GGCCGGAACA
GCCGTGGCAC GACCACCCGG GGTGGGGGCA CTTGTAGTAG CCGGCCTTGT
851 TGCTGACCCA GCTGGGCTGC ACCCTGAACT TCCCCATCAG CCCCATCGAG
ACGACTGGGT CGACCCGACG TGGGACTTGA AGGGGTAGTC GGGGTAGCTC
901 ACCGTGCCCG TGAAGCTGAA GCCCGGCATG GACGGCCCCA AGGTGAAGCA
TGGCACGGGC ACTTCGACTT CGGGCCGTAC CTGCCGGGGT TCCACTTCGT
951 GTGGCCCCTG ACCGAGGTGA AGATCAAGGC CCTGACCGCC ATCTGCGAGG
CACCGGGGAC TGGCTCCACT TCTAGTTCCG GGACTGGCGG TAGACGCTCC
1001 AGATGGAGAA GGAGGGCAAG ATCACCAAGA TCGGCCCCGA GAACCCCTAC
TCTACCTCTT CCTCCCGTTC TAGTGGTTCT AGCCGGGGCT CTTGGGGATG
1051 AACACCCCCA TCTTCGCCAT CAAGAAGGAG GACAGCACCA AGTGGCGGAA
TTGTGGGGGT AGAAGCGGTA GTTCTTCCTC CTGTCGTGGT TCACCGCCTT
1101 GCTGGTGGAC TTCCGGGAGC TGAACAAGCG GACCCAGGAC TTCTGGGAGG
CGACCACCTG AAGGCCCTCG ACTTGTTCGC CTGGGTCCTG AAGACCCTCC
1151 TGCAGCTGGG CATCCCCCAC CCCGCCGGCC TGAAGAAGAA GAAGAGCGTG
ACGTCGACCC GTAGGGGGTG GGGCGGCCGG ACTTCTTCTT CTTCTCGCAC
1201 ACCGTGCTGG ACGTGGGCGA CGCCTACTTC AGCGTGCCCC TGGACGAGGG
TGGCACGACC TGCACCCGCT GCGGATGAAG TCGCACGGGG ACCTGCTCCC
1251 CTTCCGGAAG TACACCGCCT TCACCATCCC CAGCATCAAC AACGAGACCC
GAAGGCCTTC ATGTGGCGGA AGTGGTAGGG GTCGTAGTTG TTGCTCTGGG
1301 CCGGCATCCG GTACCAGTAC AACGTGCTGC CCCAGGGCTG GAAGGGCAGC
 
GGCCGTAGGC CATGGTCATG TTGCACGACG GGGTCCCGAC CTTCCCGTCG
1351 CCCGCCATCT TCCAGGCCAG CATGACCAAG ATCCTGGAGC CCTTCCGGGC
GGGCGGTAGA AGGTCCGGTC GTACTGGTTC TAGGACCTCG GGAAGGCCCG
1401 CAAGAACCCC GAGATCGTGA TCTACCAGTA CATGGCCGCC CTGTACGTGG
GTTCTTGGGG CTCTAGCACT AGATGGTCAT GTACCGGCGG GACATGCACC
1451 GCAGCGACCT GGAGATCGGC CAGCACCGGG CCAAGATCGA GGAGCTGCGG
CGTCGCTGGA CCTCTAGCCG GTCGTGGCCC GGTTCTAGCT CCTCGACGCC
1501 GAGCACCTGC TGAAGTGGGG CTTCACCACC CCCGACAAGA AGCACCAGAA
CTCGTGGACG ACTTCACCCC GAAGTGGTGG GGGCTGTTCT TCGTGGTCTT
1551 GGAGCCCCCC TTCCTGTGGA TGGGCTACGA GCTGCACCCC GACAAGTGGA
CCTCGGGGGG AAGGACACCT ACCCGATGCT CGACGTGGGG CTGTTCACCT
1601 CCGTGCAGCC CATCCAGCTG CCCGAGAAGG ACAGCTGGAC CGTGAACGAC
GGCACGTCGG GTAGGTCGAC GGGCTCTTCC TGTCGACCTG GCACTTGCTG
1651 ATCCAGAAGC TGGTGGGCAA GCTGAACTGG ACCAGCCAGA TCTACCCCGG
TAGGTCTTCG ACCACCCGTT CGACTTGACC TGGTCGGTCT AGATGGGGCC
1701 CATCAAGGTG CGGCAGCTGT GCAAGCTGCT GCGGGGCACC AAGGCCCTGA
GTAGTTCCAC GCCGTCGACA CGTTCGACGA CGCCCCGTGG TTCCGGGACT
1751 CCGACATCGT GCCCCTGACC GAGGAGGCCG AGCTGGAGCT GGCCGAGAAC
GGCTGTAGCA CGGGGACTGG CTCCTCCGGC TCGACCTCGA CCGGCTCTTG
1801 CGGGAGATCC TGAAGGAGCC CGTGCACGGC GTGTACTACG ACCCCAGCAA
GCCCTCTAGG ACTTCCTCGG GCACGTGCCG CACATGATGC TGGGGTCGTT
1851 GGACCTGATC GCCGAGATCC AGAAGCAGGG CGACGACCAG TGGACCTACC
CCTGGACTAG CGGCTCTAGG TCTTCGTCCC GCTGCTGGTC ACCTGGATGG
1901 AGATCTACCA GGAGCCCTTC AAGAACCTGA AAACCGGCAA GTACGCCAAG
TCTAGATGGT CCTCGGGAAG TTCTTGGACT TTTGGCCGTT CATGCGGTTC
1951 CGGCGGACCA CCCACACCAA CGACGTGAAG CAGCTGACCG AGGCCGTGCA
GCCGCCTGGT GGGTGTGGTT GCTGCACTTC GTCGACTGGC TCCGGCACGT
2001 GAAGATCAGC CTGGAGAGCA TCGTGACCTG GGGCAAGACC CCCAAGTTCC
CTTCTAGTCG GACCTCTCGT AGCACTGGAC CCCGTTCTGG GGGTTCAAGG
2051 GGCTGCCCAT CCAGAAGGAG ACCTGGGAGA TCTGGTGGAC CGACTACTGG
CCGACGGGTA GGTCTTCCTC TGGACCCTCT AGACCACCTG GCTGATGACC
2101 CAGGCCACCT GGATCCCCGA GTGGGAGTTC GTGAACACCC CCCCCCTGGT
GTCCGGTGGA CCTAGGGGCT CACCCTCAAG CACTTGTGGG GGGGGGACCA
2151 GAAGCTGTGG TACCAGCTGG AGAAGGAGCC CATCGCCGGC GCCGAGACCT
CTTCGACACC ATGGTCGACC TCTTCCTCGG GTAGCGGCCG CGGCTCTGGA
2201 TCTACGTGGA CGGCGCCGCC AACCGGGAGA CCAAGATCGG CAAGGCCGGC
AGATGCACCT GCCGCGGCGG TTGGCCCTCT GGTTCTAGCC GTTCCGGCCG
2251 TACGTGACCG ACCGGGGCCG GCAGAAGATC GTGACCCTGA GCGAGACCAC
ATGCACTGGC TGGCCCCGGC CGTCTTCTAG CACTGGGACT CGCTCTGGTG
2301 CAACCAGAAA ACCGAGCTGC AGGCCATCCA GCTGGCCCTG CAGGACAGCG
GTTGGTCTTT TGGCTCGACG TCCGGTAGGT CGACCGGGAC GTCCTGTCGC
2351 AGAGCGAGGT GAACATCGTG ACCGACAGCC AGTACGCCCT GGGCATCATC
TCTCGCTCCA CTTGTAGCAC TGGCTGTCGG TCATGCGGGA CCCGTAGTAG
2401 CAGGCCCAGC CCGACCGGAG CGAGAGCGAG CTGGTGAACC AGATCATCGA
GTCCGGGTCG GGCTGGCCTC GCTCTCGCTC GACCACTTGG TCTAGTAGCT
2451 GCAGCTGATC AAGAAGGAGC GGGCCTACCT GAGCTGGGTG CCCGCCCACA
CGTCGACTAG TTCTTCCTCG CCCGGATGGA CTCGACCCAC GGGCGGGTGT
2501 AGGGCATCGG CGGCGACGAG CAGGTGGACA AGCTGGTGAG CAGCGGCATC
TCCCGTAGCC GCCGCTGCTC GTCCACCTGT TCGACCACTC GTCGCCGTAG
XbaI
-------
EcoRI
 
------
2551 CGGAAGGTGC TGTGATCTAG AGAATTC
GCCTTCCACG ACACTAGATC TCTTAAG
Sequences I.D. No: 7: Du151 synthesised Pol Protein 
121 FPQGPARQFP SEQTRANSPT SRELQVRRDN
181 PRSETGAERK GTLNFPQITL WQRPLVSIKI GGQTREALLD TGADDTVLED INLPGKWKPK
241 MIGGIGGFIK VRQYDQILIE ICGKKAIGTV LVGPTPVNII GRNMLTQLGC TLNFPISPIE
301 TVPVKLKPGM DGPKVKQWPL TEVKIKALTA ICEEMEKEGK ITKIGPENPY NTPIFAIKKE
361 DSTKWRKLVD FRELNKRTQD FWEVQLGIPH PAGLKKKKSV TVLDVGDAYF SVPLDEGFRK
421 YTAFTIPSIN NETPGIRYQY NVLPQGWKGS PAIFQASMTK ILEPFRAKNP EIVIYQYMAA
481 LYVGSDLEIG QHRAKIEELR EHLLKWGFTT PDKKHQKEPP FLWMGYELHP DKWTVQPIQL
541 PEKDSWTVND IQKLVGKLNW TSQIYPGIKV RQLCKLLRGT KALTDIVPLT EEAELZLAEN
601 REILKEPVHG VYYDPSKDLI AEIQKQGDDQ WTYQIYQEPF KNLKTGKYAK RRTTHTNDVK
661 QLTEAVQKIS LESIVTWGKT PKFRLPIQKE TWEIWWTDYW QATWIPEWEF VNTPPLVKLW
721 YQLEKEPIAG AETFYVDGAA NRETKIGKAG YVTDRGRQKI VTLSETTNQK TELQAIQLAL
781 QDSESEVNIV TDSQYALGII QAQPDRSESE LVNQIIEQLI KKERAYLSWV PAHKGIGGDE
841 QVDKLVSSGI RKVL*
Sequence I.D. No: 8: Du151 synthesised *env* Gene 
1 ATGA GGGTTATGGG GATTCAGAGA AACTGGCCTC AGTGGTGGAT TTGGGGGACA
61 TTGGGATTTT GGATGATCAT CATCTGTCGC GTCGTGGGCA ACCTGAACCT GTGGGTCACT
121 GTCTACTATG GAGTGCCAGT TTGGAAGGAA GCCAAGACAA CTCTGTTTTG CGCCAGCGAC
181 GCCAAGGCTT ATGACAAGGA AGTCCACAAC GTGTGGGCCA CCCACGCATG TGTCCCAACC
241 GACCCCAACC CACGCGAAAT CGTGCTGGAA AACGTCACAG AAAATTTCAA CATGTGGAAA
301 AACGATATGG TGGATCAGAT GCATGAGGAT ATTATTAGCC TCTGGGACCA GTCTCTGAAG
361 CCATGTGTGA AGTTGACACC TCTCTGTGTG ACCCTTAACT GTACTAACGC CCCCGCCTAT
421 AACAACTCTA TGCACGGGGA GATGAAAAAC TGTTCCTTCA ACACCACCAC CGAAATCAGG
481 GACAGAAAAC AGAAAGCCTA TGCCCTGTTC TATAAGCCCG ATGTGGTGCC ACTTAACCGC
541 CGCGAAGAAA ATAATGGTAC TGGCGAATAT ATTCTGATTA ACTGTAACAG CTCTACAATT
601 ACTCAGGCTT GCCCTAAAGT CACCTTTGAC CCAATCCCAA TCCACTACTG CGCCCCTGCA
661 GGATACGCTA TCCTGAAATG CAATAATAAG ACCTTCAACG GAACTGGACC CTGCAATAAC
721 GTGTCTACAG TGCAATGTAC CCACGGCATT ATGCCCGTCG TCTCCACCCA ACTGCTGCTC
781 AATGGCAGCT TGGCAGAAGA GGAGATCATT ATTAGGAGCG AAAACCTCAC CAACAATATC
841 AAGACAATCA TCGTGCACCT GAACAAGTCT GTGGAAATTG TGTGTACCAG GCCCAATAAC
901 AACACCAGGA AGAGCATCCG CATCGGACCT GGACAAACTT TCTACGCCAC CGGCGAAATC
961 ATCGGGAACA TTAGAGAAGC CCACTGCAAC ATCTCTAAGA GCAATTGGAC ATCTACATTG
1021 GAGCAAGTGA AAAAAAAGCT GAAAGAGCAC TACAATAAGA CCATCGAGTT CAACCCTCCT
1081 TCCGGCGGCG ATCTGGAGGT CACAACACAC TCCTTTAACT GTAGGGGGGA GTTCTTTTAC
1141 TGCAACACAA CAAAGCTGTT TAGCAACAAC TCCGACAGCA ATAATGAGAC TATCACCCTG
1201 CCTTGCAAGA TCAAGCAAAT CATTAACATG TGGCAGAAAG TGGCAAGGCC AATGTATGCA
1261 CCTCCCATCG AGGGCAACAT CACATGCAAG TCTAATATCA CCGGCCTGTT GCTGACTAGA
1321 GACGGTGGCA AGAATACTAC TAACGAAATC TTCAGGCCAG GTGGAGGGAA CATGAAAGAT
1381 AATTGGCGCT CCGAACTGTA TAAGTACAAG GTGGTGGAGA TTGAGCCCCT CGGCGTCGCC
1441 CCCACAAAGT CTAAGCGCCG CGTGGTGGAA AGAGAGAAGA GGGCTGTCGG CCTCGGCGCA
1501 GTGCTGCTGG GGTTCTTGGG TGCCGCTGGG TCTACAATGG GCGCTGCCTC TATTACACTC
1561 ACCGTGCAAG CTAGGCAGCT GCTGTCCGGT ATTGTGCAAC AACAGAGCAA TCTCTTGAGA
1621 GCTATCGAGG CCCAGCAGCA TATGCTGCAA CTTACAGTGT GGGGTATTAA GCAGCTGCAA
1681 ACTCGCGTCC TGGCAATCGA ACGCTACCTG AAAGACCAGC AACTCCTGGG TCTGTGGGGC
1741 TGCTCCGGTA AGATCATCTG TACCACAGCC GTGCCCTGGA ACAGCAGCTG GTCCAATAAG
1801 AGCCAAGAGG ATATTTGGGA TAATATGACC TGGATGCAAT GGGATAGAGA GATCAGCAAC
1861 TACACAGGAA CCATTTATAG GCTCCTGGAA GATTCTCAGA ACCAGCAGGA GAAGAACGAG
1921 AAGGACTTGC TCGCCCTGGA TAGCTGGAAA AACCTGTGGA ATTGGTTTAA CATCACCAAC
1981 TGGCTTTGGT ACATTAAGAT TTTCATCATG ATTGTGGGAG GCTTGATCGG CCTGAGGATT
2041 ATCTTCGGGG TGCTTGCCAT TGTGAAAAGG GTCAGACAAG GATACTCCCC ATTGTCCTTT
 
2101 CAGACCTTGA CTCCAAGCCC ACGCGGACCC GACAGGTTGG GCAGGATCGA GGAGGAAGGA
2161 GGCGAACAGG ATAAGGACCG CTCCATCAGA CTTGTTAGCG GGTTTCTGGC CCTGGCCTGG
2221 GATGATCTGA GGAGCCTGTG CCTCTTCTCC TATCACCACC TCCGCGATTT CATCCTCATT
2281 GCAGCTAGGG CTGCTGAGTT GCTGGCACGC TCCTCCCTGA GAGGTCTCCA GAGAGGCTGG
2341 GAGGCACTGA AGTACCTCGG GAACCTTGTG CAATACGGCG GGCTGGAGCT GAAAAGATCC
2401 GCCATCAAGC TGTTCGACAC CATCGCAATC GCCGTTGCAG AGGGCACCGA CAGGATCTTG
2461 GAGGTCATTC AGAGGATCTG TCGCGCCATC CGCCACATCC CCATCAGGAT CAGACAAGGA
2521 TTCGAGGCAG CACTGCAATG ATAGTTAATT AA
Sequence I.D. No: 9: Du151 synthesised Env Protein 
MRVMGIQRNWPQWWIWGTLGFWMIIICRVVGNLNLWVTVYYGVPVWKEAKTTLFCASD
AKAYDKEVHNVWATHACVPTDPNPREIVLENVTENFNMWKNDMVDQMHEDIISLWDQSLK
PCVKLTPLCVTLNCTNAPAYNNSMHGEMKNCSFNTTTEIRDRKQKAYALFYKPDVVPLNR
REENNGTGEYILINCNSSTITQACPKVTFDPIPIHYCAPAGYAILKCNNKTFNGTGPCNN
VSTVQCTHGIMPVVSTQLLLNGSLAEEEIIIRSENLTNNIKTIIVHLNKSVEIVCTRPNN
NTRKSIRIGPGQTFYATGEIIGNIREAHCNISKSNWTSTLEQVKKKLKEHYNKTIEFNPP
SGGDLEVTTHSFNCRGEFFYCNTTKLFSNNSDSNNETITLPCKIKQIINMWQKVGRAMYA
PPIEGNITCKSNITGLLLTRDGGKNTTNEIFRPGGGNMKDNWRSELYKYKVVEIEPLGVA
PTKSKRRVVEREKRAVGLGAVLLGFLGAAGSTMGAASITLTVQARQLLSGIVQQQSNLLR
AIEAQQHMLQLTVWGIKQLQTRVLAIERYLKDQQLLGLWGCSGKIICTTAVPWNSSWSNK
SQEDIWDNMTWMQWDREISNYTGTIYRLLEDSQNQQEKNEKDLLALDSWKNLWNWFNITN
WLWYIKIFIMIVGGLIGLRIIFGVLAIVKRVRQGYSPLSFQTLTPSPRGPDRLGRIEEEG
GEQDKDRSIRLVSGFLALAWDDLRSLCLFSYHHLRDFILIAARAAELLGRSSLRGLQRGW
EALKYLGNLVQYGGLELKRSAIKLFDTIAIAVAEGTDRILEVIQRICRAIRHIPIRIRQG
FEALQ
Sequence I.D. No: 10: Du179 Env Gene (non-humanised) 
ATGAGAGT
GAGGGGGATACAGAGGAATTGGCCACAATGGTGGATATGGGGCATCTTAGGCTTTTGGATGTTAATGATTTGTAGTGGG
GTGGGAAACTTGTGGGTCACAATCTATTATGGGGTACCTGTGTGGAGAGAAGCAAAAACTACTCTATTCTGTGCATCAG
ATGCTAAAGCATATGATAGAGAAGTGCATAATGTCTGGGCTACACATGCCTGTGTACCCACAGACCCCAACCCACAAGA
AATAGTTATGGGAAATGTAACAGAAAATTTTAACATGTGGAAAAATGACATGGTGGATCAGATGCATGAGGATATAATC
AATTTATGGGATCAAAGCCTAAAGCCATGTGTAAAGTTAACCCCACTCTGTGTCACTTTAAAATGTAGTACCTATAATG
GTAGTGATACCAACGATATGAGAAATTGCTCTTTCAATACAACTACAGAAATAAGGGACAAGAAACAGACAGTGTATGC
ACTTTTTTATAAACCTGATATAGTACCAATTAATGAGAGTGAGTATATATTAATACATTGCAATACCTCAACCATAACA
CAAGCCTGTCCAAAGGTCTCTTTTGACCCAATTCCTATACATTATTGTGCTCCAGCTGGTTATGCGATTCTAAAGTGTA
ATAATAAGACATTCAATGGACGGGACCATGCCAAAATGTCAGCACAGTACAATGCACACATGGAATTAAGCCAGTAGT
ATCAACTCAACTACTGTTAAATGGTAGCATAGCAGAAGGAGAGATAATAATTAGATCTGAAAATCTGACAAACAATGTT
AAAACAATAATAGTACACCTTAATGAATCTATAGGAATTGTGTGTACAAGACCCGGCAATAATACAAGAAAAAGTATAA
GGATAGGACCAGGACAAGCATTCTATACAAATCACATAATAGGAGATATAAGACAAGCATATTGTAACATTAGTAAACA
AGAATGGAACAAAACTTTAGAAGAGGTGAGAAAAAAATTGCAAGAACACTTCCCAAATAAAACAATAAAATTTAACTCA
TCCTCAGGAGGGGACCTAGAAATTACAACACATAGCTTTAATTGCAGAGGAGAATTTTTCTATTGCAATACATCAAAAC
TATTTAATGATAGTCTAGTAAATGATACAGAAAGTAATTCAACCATCACTATTCCATGCAGAATAAAACAAATTATAAA
CATGTGGCAGGAGGTAGGACGAGCAATGTATGCCCCTCCCATTGCAGGAAACATAACATGTAAATCAAATATCACAGGA
CTACTATTGACACGTGATGGAGGAACAGATAACACAACAGAGATATTCAGACCTGGAGGAGGAAATATGAAGGACAATT
GGAGAAGTGAATTATATAAATATAAAGTAGTAGAAATTAAGCCATTGGGAATAGCACCCACTGAAGCAAAAAGGAGAGT
GGTGGAGAGAGAAAAAAGAGCAGTGGGAATAGGAGCTGTGCTCCTTGGGTTCTTGGGAGCAGCAGGAAGCACTATGGGC
GCGGCGTCAATAACGCTGACGGTACAGGCCAGACAACTGTTGTCTGGTATAGTGCAACAGCAAAGCAATTTGCTGAGAG
CTATAGAGGCGCAACAGCATATGTTGCAACTCACAGTCTGGGGCATTAAGCAGCTCCAGACAAGAGTCCTGGCTATAGA
AAGATACCTAAAGGATCAACAGCTCCTAGGACTTTGGGGCTGCTCTGGAAAACTCATCTGCACCACTAATGTGCCTTGG
AACTCCAGTTGGAGCAATAAATCTCAACAAGCTATTTGGGATAACATGACATGGATGCAGTGGGATAGAGAAATTAATA
ATTACACAAACATAATATACCAGTTGCTTGAGGACTCGCAAATCCAGCAGGAACAGAATGAAAAAGATTTATTAGCATT
GGACAAGTGGCAAAATCTGTGGAGTTGGTTTAGCATAACAAATTGGCTATGGTATATAAAAATATTCATAATGATAGTA
GGAGGCTTAATAGGTTTAAGAATAATTTTTGCTGTGCTATCTATAGTAAATAGAGTTAGGCAGGGATACTCACCTTTGT
CGTTTCAGACCCTTACCCCAAACCCGAGGGGACCCGACAGGCTCGGAGAAATCGAAGAAGAAGGTGGAGAGCAAGACAG
 
AGACAGATCCGTTCGATTAGTGAGCGGATTCTTACCACTTGCCTGGGACGATCTGCGGAGCCTGTGCCTCTTCAGCTAC
CACCGATTGAGAGACTTCATATTCGATTGCAGCGAGGACAGTGGAACTTCTGGGACGCAGCAGTCTCAGGGGACTCCAG
AGGGGTGGGAACTCCTTAAATATCTGGAAGCCTTGTGCAGTATTGGGGTCTGGAGCTAAAAAGAGTGCTATTAGTCTG
CTTGATACCCATAGCAATAGCAGTAGCTGAAGGAACAGATAGGCATTATTGAATTAGTACTAAGATTTTGTAGAGCTATC
CGCAACATACCTACAAGAGTAAGACAGGGCTGTGAAGCAGCTTTGCTATAA
Sequence I.D. No: 11: Du179 Env Protein 
1 MRVRGIQ RNWPQWWIWG ILGFWMLMIC SGVGNLWVTI
61 YYGVPVWREA KTTLFCASDA KAYDREVHNV WATHACVPTD PNPQEIVMGN VTENFNMWKN
121 DMVDQMHEDI INLWDQSLKP CVKLTPLCVT LKCSTYNGSD TNDMRNCSFN TTTEIRDKKQ
181 TVYALFYKPD IVPINESEYI LIHCNTSTIT QACPKVSFDP IPIHYCAPAG YAILKCNNKT
241 FNGTGPCQNV STVQCTHGIK PVVSTQLLLN GSIAEGEIII RSENLTNNVK TIIVHLNESI
301 GIVCTRPGNN TRKSIRIGPG QAFYTNHIIG DIRQAYCNIS KQEWNKTLEE VRKKLQEHFP
361 NKTIKFNSSS GGDLEITTHS FNCRGEFFYC NTSKLFNDSL VNDTESNSTI TIPCRIKQII
421 NMWQEVGRAM YAPPIAGNIT CKSNITGLLL TRDGGTDNTT EIFRPGGGNM KDNWRSELYK
481 YKVVEIKPLG IAPTEAKRRV VEREKRAVGI GAVLLGFLGA AGSTMGAASI TLTVQARQLL
541 SGIVQQQSNL LRAIEAQQHM LQLTVWGIKQ LQTRVLAIER YLKDQQLLGL WGCSGKLICT
601 TNVPWNSSWS NKSQQAIWDN MTWMQWDREI NNYTNIIYQL LEDSQIQQEQ NEKDLLALDK
661 WQNLWSWFSI TNWLWYIKIF IMIVGGLIGL RIIFAVLSIV NRVRQGYSPL SFQTLTPNPR
721 GPDRLGEIEE EGGEQDRDRS VRLVSGFLPL AWDDLRSLCL FSYHRLRDFI FDCSEDSGTS
781 GTQQSQGTPE GWEVLKYLGS LVQYWGLELK RVLLVCLIPI AIAVAEGTDR IIELVLRFCR
841 AIRNIPTRVR QGCEAALL*
SEQUENCE I.D. No. 12: Consensus amino acid sequence for the partial *gag* gene of HIV-1 subtype C 
GEKLDKWEKI RLRPGGKKHY MLKHLVWASR ELERFALNPG LLETSEGCKQ 50
IMKQLQPALQ TGTEELRSLY NTVATLYCVH EKIEVRDTKE ALDKIEEEQN 100
KSQQ-CQQKT QQAKAADGG- KVSQNYPIVQ NLQGQMVHQA ISPRTLNAWV 150
EEKAFSP EVIPMFTALS EGATPQDLNT MLNTVGGHQA AMQMLKDTIN 200
EEAAEWDRLH PVHAGPIAPG QMREPRGSDI AGTTSTLQEQ IAWMTSNPPI 250
PVGDIYKRWI ILGLNKIVRM YSPVSILDIK QGPKEPFRDY VDRFFKTLRA 300
EQATQDVKNW MTD 313
SEQUENCE I.D. No. 13 : Consensus amino acid sequence for the partial *pol* gene of HIV-1 subtype C 
LTEEKIKALT AICEEMEKEG KITKIGPENP YNTPVFAIKK KDSTKWRKL- 50
VDFRELNKRT QDFWEVQLGI PHPAGLKKKK SVTVLDVGDA YFSVPLDEGF 100
RKYTAFTIPS INNETPGIRY QYNVLPQGWK GSPAIFQSSM TKILEPFRAK 150
NPEIVIYQYM DDLYVGSDLE IGQHRAKIEE LREHLLKWGF TTPDKKHQKE 200
PPFLWMGYEL HPDKWTVQPI QLPEKDSWTV NDIQKLVGKL NWASQIYPGI 250
KVRQLCKLLR GAKALTDIVP LTEEAELE 278
SEQUENCE I.D. No. 14 : Consensus amino acid sequence for the partial *env* gene of HIV-1 subtype C

YCAPAGYAIL KCNNKTFNGT GPCNNVSTVQ CTHGIKPVVS TQLLLNGSLA 50
EEEIIIRSEN LTNNAKTIIV HLNESVEIVC TRPNNNTRKS IRIGPGQIFY 100
ATGDIIGDIR QAHCNISEGK WNKTLQKVKK KLKEELYKYK VVEIKPLGIA 150
PTEAKRRVVE REKRAVGIGA VFLGFLGAAG STMGAASITL TVQARQLLSG 200
IVQQQSNLLR AIEAQQHMLQ LTVWGIKQL 229

## Claims

1. A process for the selection of HIV subtype isolates for use in a vaccine comprising the following steps:
(a) isolating viruses from recently infected subjects;
(b) comparing the sequence, or part of the sequence, of at least one HIV gene of the
isolated viruses, and generating a consensus amino acid sequence by identifying the most common codon or amino acid among the isolated viruses at each position along said sequence;
(c) selecting among all the isolated viruses the strains that fulfill the following criteria:
(i) that the strain be genotypically representative of the circulating strains;
(ii) that the strain not be an outlier strain;
(iii) that the strain be as close as possible to the consensus, amino acid sequence of (b);
(iv) that the strains have an R5 phenotype, ie a phenotype associated with transmission for selection of the RNA or cDNA to be included for the *env* region; and
(v) that the strain be able to be grown in tissue culture.

2. A process according to claims 1, Wherein the HIV subtype is HIV-1 subtype C.

3. A process according to claim 2, wherein the phenotype which is associated with transmission is a virus that utilises the CCRS co-receptor and is non syncitium inducing, (NSI).

4. A process according to anyone of claims 1 to 3, wherein the selected isolate is an HIV-1 subtype C isolate, designated Du422 and assigned, Accession Number 01032114 by the European Collection of Cell Cultures.

5. A process according to any one of claims 1 to 3, wherein the selected isolate is an HIV-1 subtype C. isolate, designated Du151 and assigned Accession Number 00072724 by the European Collection of Cell Cultures.

6. A process according to any one of claims 1 to 3, wherein the selected isolate has:
(i) the nucleotide sequence of the gag gene of HIV-1 subtype C isolate Du422, assigned Accession Number 01032114 by the European Collection of Cell Cultures;
(ii) an RNA sequence corresponding to the nucleotide sequence set out in (i); or
(iii) the sequence set out in Sequence I.D. No 4.

7. A process according to any one of claims 1 to 5, wherein the selected isolate has:
(i) a nucleotide sequence of the *pol* gene of HIV-1 subtype C isolate Du151 assigned Accession Number 00072724 by the European Collection of Cell Cultures;
(ii) an RNA sequence corresponding to the nucleotide sequence set out in (i); or
(iii) the sequence set out in Sequence I.D. No. 6.

8. A process according to any one of claims 1 to 3, wherein the selected isolate has:
(i) a nucleotide sequence of the *env* gene of HIV-1 subtype C isolate Du151 assigned Accession Number 00072724 by the European Collection of Cell Cultures;
(ii) an RNA sequence corresponding to the nucleotide sequence set out in (i); or
(iii) the sequence set out in Sequence I.D. No. 8.

9. A process according to any one of claims 1 to 3, wherein the selected isolate has:
(i) the amino acid sequence set out in Sequence I.D. No.1;
(ii) the sequence set out in Sequence I.D**.** No. 5; or
(iii) the amino acid sequence encoded by the nucleotide sequence of the *gag* gene of HIV-1 subtype C isolate Du422 (European Collection of Cell Cultures Accession Number 010321114).

10. A process according to any one of claims 1 to 3, wherein the selected isolate has:
(i) the amino acid sequence set out in Sequence I.D. No. 2;
(ii) the sequence set out in Sequence I.D. No. 7; or
(iii) the amino acid sequence encoded by the nucleotide sequence of the *pol* gene of HIV-1 subtype C isolate Du151 (European Collection of Cell Cultures Accession Number 0007272).

11. A process according to any one of claims 1 to 3, wherein the selected isolate has:
(i) the amino acid sequence set out in Sequence I.D. No. 3;
(ii) the sequence set out in Sequence I.D. No. 9; or
(iii) the amino acid sequence encoded by the nucleotide sequence of the *env* gene of HIV-1 subtype C isolate Du151 (European Collection of Cell Cultures Accession Number 00072724).

12. A. pharmaceutical composition comprising an HIV-1 subtype C isolate or a nucleic acid encoding a *gag*, *pol* or *env* gene thereof, wherein the isolate is an isolate defined in anyone of claims 4 or 5, or wherein the isolate is selected according to the process of anyone of claims 1 to 11 and has a sequence set out in any one of claims 6 to 11.

13. An HIV-1 subtype isolate selected according to the process of any one of claims 1 to 11, or a nucleic acid encoding a *gag*, *pol* or *env* gene thereof, for use in therapy, wherein the isolate is an isolate defined in anyone of claims 4 or 5, or wherein the isolate is selected according to the process of anyone of claims 1 to 11 and has a sequence set out in any one of claims 6 to 11.

14. The use of an HIV-1 subtype isolate selected according to the process of any one of claims 1 to 11 or a nucleic acid encoding a *gag*, *pol* or *env* gene thereof in the preparation of a vaccine, wherein the isolate is an isolate defined in anyone of claims 4 or 5, or wherein the isolate is selected according to the process of anyone of claims 1 to 11 and has a sequence set out in any one of claims 6 to 11.

## Patentansprüche

1. Verfahren zur Selektion von HIV Subtyp-Isolaten zur Verwendung in einem Impfstoff, umfassend die folgenden Schritte:
(a) Isolieren von Viren von kürzlich infizierten Patienten;
(b) Vergleichen der Sequenz, oder eines Teils der Sequenz, von mindestens einem HIV-Gen der isolierten Viren, und Generieren einer Konsens-Aminosäuresequenz durch Identifizieren des üblichsten Codons oder der üblichsten Aminosäure an jeder Position entlang dieser Sequenz unter den isolierten Viren;
(c) Selektieren der Stämme unter allen isolierten Viren, die die folgenden Kriterien erfüllen:
(i) dass der Stamm genotypisch repräsentativ für die zirkulierenden Stämme ist;
(ii) dass der Stamm nicht ein Ausreisserstamm ist;
(iii) dass der Stamm so nah wie möglich zu der Konsens-Aminosäuresequenz aus (b) ist;
(iv) dass der Stamm einen R5-Phänotyp hat, d.h. einen Phänotyp, der mit der Übertragung zur Selektion der RNS oder cDNS, die für die *env-*Region eingefügt werden soll, assoziiert ist; und
(v) dass der Stamm in Gewebekultur gezüchtet werden kann.

2. Verfahren nach Anspruch 1, wobei der HIV-Subtyp HIV-1 Subtyp C ist.

3. Verfahren nach Anspruch 2, wobei der Phänotyp, der mit Übertragung assoziiert ist, ein Virus ist, der den CCR5-Corezeptor nutzt und nicht Synzytium-induzierend (non syncitium inducing, NSI) ist.

4. Verfahren nach einem der Ansprüche 1 bis 3, wobei das selektierte Isolat ein HIV-1 Subtyp C-Isolat ist, das mit Du422 bezeichnet wird und dem die Zugangsnummer 01032114 (Accession Number) von der European Collection of Cell Culture zugeteilt ist.

5. Verfahren nach einem der Ansprüche 1 bis 3, wobei das selektierte Isolat ein HIV-1 Subtyp C-Isolat ist, das mit Du151 bezeichnet wird und dem die Zugangsnummer 00072724 (Accession Number) von der European Collection of Cell Culture zugeteilt ist.

6. Verfahren nach einem der Ansprüche 1 bis 3, wobei das selektierte Isolat aufweist:
(i) die Nukleotidsequenz des *gag*-Gens des HIV-1 Subtyp C-Isolats Du422, dem die Zugangsnummer 01032114 von der European Collection of Cell Culture zugeteilt ist;
(ii) eine RNS-Sequenz, die der in (i) aufgezeigten Nukleotidsequenz entspricht; oder
(iii) eine Sequenz, die in Sequenz I.D. Nr. 4 aufgezeigt ist.

7. Verfahren nach einem der Ansprüche 1 bis 3, wobei das selektierte Isolat aufweist:
(i) eine Nukleotidsequenz des *pol*-Gens des HIV-1 Subtyp C-Isolats Du151, dem die Zugangsnummer 00072724 von der European Collection of Cell Culture zugeteilt ist;
(ii) eine RNS-Sequenz, die der in (i) aufgezeigten Nukleotidsequenz entspricht; oder
(iii) eine Sequenz, die in Sequenz I.D. Nr. 6 aufgezeigt ist.

8. Verfahren nach einem der Ansprüche 1 bis 3, wobei das selektierte Isolat aufweist:
(i) eine Nukleotidsequenz des *env*-Gens des HIV-1 Subtyp C-Isolats Du151, dem die Zugangsnummer 00072724 von der European Collection of Cell Culture zugeteilt ist;
(ii) eine RNS-Sequenz, die der in (i) aufgezeigten Nukleotidsequenz entspricht; oder
(iii) eine Sequenz, die in Sequenz I.D. Nr. 8 aufgezeigt ist.

9. Verfahren nach einem der Ansprüche 1 bis 3, wobei das selektierte Isolat aufweist:
(i) die Aminosäuresequenz, die in Sequenz I.D. Nr. 1 aufgezeigt ist;
(ii) die Sequenz, die in Sequenz I.D. Nr. 5 aufgezeigt ist; oder
(iii) die Aminosäuresequenz, die durch die Nukleotidsequenz des *gag*-Gens des HIV-1 Subtyp C-Isolats Du422 (European Collection of Cell Cultures Zugangsnummer 01032114) kodiert ist.

10. Verfahren nach einem der Ansprüche 1 bis 3, wobei das selektierte Isolat aufweist:
(i) die Aminosäuresequenz, die in Sequenz I.D. Nr. 2 aufgezeigt ist;
(ii) die Sequenz, die in Sequenz I.D. Nr. 7 aufgezeigt ist; oder
(iii) die Aminosäuresequenz, die durch die Nukleotidsequenz des *pol*-Gens des HIV-1 Subtyp C-Isolats Du151 (European Collection of Cell Cultures Zugangsnummer 0007272) kodiert ist.

11. Verfahren nach einem der Ansprüche 1 bis 3, wobei das selektierte Isolat aufweist:
(i) die Aminosäuresequenz, die in Sequenz I.D. Nr. 3 aufgezeigt ist;
(ii) die Sequenz, die in Sequenz I.D. Nr. 9 aufgezeigt ist; oder
(iii) die Aminosäuresequenz, die durch die Nukleotidsequenz des *env*-Gens des HIV-1 Subtyp C-Isolats Du151 (European Collection of Cell Cultures Zugangsnummer 00072724) kodiert ist.

12. Pharmazeutische Zusammensetzung, umfassend ein HIV-1 Subtyp C-Isolat oder eine Nukleinsäure, die ein *gag-, pol-* oder *env-*Gen davon kodiert, wobei das Isolat ein in einem der Ansprüche 4 oder 5 definiertes Isolat ist, oder wobei das Isolat gemäß dem Verfahren nach einem der Ansprüche 1 bis 11 selektiert ist und eine Sequenz aufweist, die in einem der Ansprüche 6 bis 11 aufgezeigt ist.

13. HIV-1 Subtyp-Isolat, selektiert gemäß dem Verfahren nach einem der Ansprüche 1 bis 11, oder Nukleinsäure, die ein *gag-, pol-* oder *env*-Gen davon kodiert, zur Verwendung bei der Therapie, wobei das Isolat ein in einem der Ansprüche 4 oder 5 definiertes Isolat ist, oder wobei das Isolat gemäß dem Verfahren nach einem der Ansprüche 1 bis 11 selektiert ist und eine Sequenz aufweist, die in einem der Ansprüche 6 bis 11 aufgezeigt ist.

14. Verwendung eines HIV-1 Subtyp-Isolats, selektiert gemäß dem Verfahren nach einem der Ansprüche 1 bis 11, oder einer Nukleinsäure, die ein *gag*-, *pol*- oder *env*-Gen davon kodiert, bei der Herstellung eines Impfstoffs, wobei das Isolat ein in einem der Ansprüche 4 oder 5 definiertes Isolat ist, oder wobei das Isolat gemäß dem Verfahren nach einem der Ansprüche 1 bis 11 selektiert ist und eine Sequenz aufweist, die in einem der Ansprüche 6 bis 11 aufgezeigt ist.

## Revendications

1. Processus de sélection d'isolats d'un sous-type du VIH à utiliser pour un vaccin comprenant les étapes suivantes :
(a) isoler des virus à partir de sujets récemment infectés ;
(b) comparer la séquence, ou une partie de la séquence, d'au moins un gène du VIH des virus isolés, et générer une séquence consensus d'acides aminés en identifiant l'acide aminé ou codon le plus commun parmi les virus isolés à chaque position le long de ladite séquence ;
(c) sélectionner parmi tous les virus isolés les souches qui répondent aux critères suivants :
(i) que la souche soit génotypiquement représentative des souches en circulation ;
(ii) que la souche ne soit pas une souche marginale ;
(iii) que la souche soit la plus proche possible de la séquence consensus d'acides aminés de (b) ;
(iv) que la souche possède un phénotype R5, c'est-à-dire, un phénotype associé à la transmission pour la sélection de l'ARN ou ADN complémentaire à inclure pour la région *env* ; et
(v) que la souche soit apte à croître dans une culture tissulaire.

2. Processus selon la revendication 1, dans lequel le sous-type du VIH est le sous-type C du VIH-1.

3. Processus selon la revendication 2, dans lequel le phénotype qui est associé à la transmission est un virus qui utilise le corécepteur CCR5 et est non inducteur de syncytium (NSI).

4. Processus selon l'une quelconque des revendications 1 à 3, dans lequel l'isolat sélectionné est un isolat de sous-type C du VIH-1, désigné par Du422 et disposant du Numéro d'Entrée 01032114 auprès de la European Collection of Cell Cultures.

5. Processus selon l'une quelconque des revendications 1 à 3, dans lequel l'isolat sélectionné est un isolat de sous-type C du VIH-1, désigné par Du151 et disposant du Numéro d'Entrée 00072724 auprès de la European Collection of Cell Cultures.

6. Processus selon l'une quelconque des revendications 1 à 3, dans lequel l'isolat sélectionné a :
(i) la séquence des nucléotides du gène *gag* de l'isolat Du422 de sous-type C du VIH-1, disposant du Numéro d'Entrée 01032114 auprès de la European Collection of Cell Cultures ;
(ii) une séquence d'ARN correspondant à la séquence des nucléotides selon (i) ; ou
(iii) la séquence selon la Séquence I.D. No.4.

7. Processus selon l'une quelconque des revendications 1 à 3, dans lequel l'isolat sélectionné a :
(i) une séquence des nucléotides du gène *pol* de l'isolat Du151 de sous-type C du VIH-1, disposant du Numéro d'Entrée 00072724 auprès de la European Collection of Cell Cultures ;
(ii) une séquence d'ARN correspondant à la séquence des nucléotides selon (i) ; ou
(iii) la séquence selon la Séquence I.D. No.6.

8. Processus selon l'une quelconque des revendications 1 à 3, dans lequel l'isolat sélectionné a :
(i) une séquence des nucléotides du gène *env* de l'isolat Du151 de sous-type C du VIH-1, disposant du Numéro d'Entrée 00072724 auprès de la European Collection of Cell Cultures ;
(ii) une séquence d'ARN correspondant à la séquence des nucléotides selon (i) ; ou
(iii) la séquence selon la Séquence I.D. No.8.

9. Processus selon l'une quelconque des revendications 1 à 3, dans lequel l'isolat sélectionné a :
(i) la séquence d'acides aminés selon la Séquence I.D. No. 1 ;
(ii) la séquence selon la Séquence I.D. No.5 ; ou
(iii) la séquence d'acides aminés codée par la séquence des nucléotides du gène *gag* de l'isolat Du422 de sous-type C du VIH-1 (Numéro d'Entrée 01032114 auprès de la European Collection of Cells Culture).

10. Processus selon l'une quelconque des revendications 1 à 3, dans lequel l'isolat sélectionné a :
(i) la séquence d'acides aminés selon la Séquence I.D.
No.2 ;
(ii) la séquence selon la Séquence I.D. No.7 ; ou
(iii) la séquence d'acides aminés codée par la séquence des nucléotides du gène *pol* de l'isolat Du151 de sous-type C du VIH-1 (Numéro d'Entrée 0007272 auprès de la European Collection of Cells Culture).

11. Processus selon l'une quelconque des revendications 1 à 3, dans lequel l'isolat sélectionné a :
(i) la séquence d'acides aminés selon la Séquence I.D.
No.3 ;
(ii) la séquence selon la Séquence I.D. No.9 ; ou
(iii) la séquence d'acides aminés codée par la séquence des nucléotides du gène *env* de l'isolat Du151 de sous-type C du VIH-1 (Numéro d'Entrée 00072724 auprès de la European Collection of Cells Culture).

12. Composition pharmaceutique comprenant un isolat de sous-type C du VIH-1 ou un acide nucléique codant un gène *gag, pol* ou *env* correspondant, dans laquelle l'isolat est un isolat selon l'une quelconque des revendications 4 ou 5, ou dans laquelle l'isolat est sélectionné selon le processus de l'une quelconque des revendications 1 à 11 et a une séquence selon l'une quelconque des revendications 6 à 11.

13. Isolat d'un sous-type du VIH-1 sélectionné selon le processus de l'une quelconque des revendications 1 à 11 ou un acide nucléique codant un gène *gag, pol* ou *env* correspondant à utiliser dans une thérapie, dans lequel l'isolat est un isolat selon l'une quelconque des revendications 4 ou 5, ou dans lequel l'isolat est sélectionné selon le processus de l'une quelconque des revendications 1 à 11 et a une séquence selon l'une quelconque des revendications 6 à 11.

14. Utilisation d'un isolat d'un sous-type du VIH-1 sélectionné selon le processus de l'une quelconque des revendications 1 à 11 ou un acide nucléique codant un gène *gag, pol* ou *env* correspondant dans la préparation d'un vaccin, dans laquelle l'isolat est un isolat selon l'une quelconque des revendications 4 ou 5, ou dans laquelle l'isolat est sélectionné selon le processus de l'une quelconque des revendications 1 à 11 et a une séquence selon l'une quelconque des revendications 6 à 11.
